# EUROPEAN PATENT SPECIFICATION

(11) **EP 4 313 026 B1**
(45) Date of publication and mention of the grant of the patent: **03.09.2025**
(21) Application number: 22721524.1
(22) Date of filing: 01.04.2022
(51) Int. Cl.: A61K 31/341, A61P 7/10, A61P 9/04, A61P 9/12, A61K 9/00, A61K 9/08, A61K 47/18

(54) **SUBCUTANEOUS INFUSION OF FUROSEMIDE**
SUBKUTANE INFUSION VON FUROSEMID
INFUSION SOUS-CUTANÉE DE FUROSÉMIDE

(30) Priority: 02.04.2021 US 202163169998 P
(43) Date of publication of application: 07.02.2024
(73) Proprietor: scPharmaceuticals Inc., Burlington, MA 01803 (US)
(72) Inventor: MOHR, John, F., College Station, TX 77845 (US); TUCKER, John, Rye, NH 03871 (US); CORNELIUS, Barbara, Westford, 01886 (US); PATEL, Bhavini, Burlington, MA 01803 (US); KAMINENI, Phani, Waltham, MA 02451 (US)
(74) Representative: Graham Watt & Co LLP
(86) International application number: PCT/US2022/023003
(87) International publication number: WO 2022/212813

(56) References cited:
- WO-A1-2022/031959
- NCT04161482: "An Open Label Study to Evaluate the Impact of Duration of Subcutaneous Infusion of a Novel, pH Neutral Formulation of Furosemide (Furoscix ) on Safety and Local Skin Tolerability", CLINICALTRIALS.GOV, 10 July 2020 (2020-07-10), XP055935978, Retrieved from the Internet <URL:https://clinicaltrials.gov/ct2/show/NCT04161482> [retrieved on 20220628]
- DOMENIC A. SICA ET AL: "Subcutaneous Furosemide in Heart Failure", JACC: BASIC TO TRANSLATIONAL SCIENCE, vol. 3, no. 1, 1 February 2018 (2018-02-01), pages 25 - 34, XP055675008, ISSN: 2452-302X, DOI: 10.1016/j.jacbts.2017.10.001
- GILOTRA NISHA A. ET AL: "Efficacy of Intravenous Furosemide Versus a Novel, pH-Neutral Furosemide Formulation Administered Subcutaneously in Outpatients With Worsening Heart Failure", JACC: HEART FAILURE, vol. 6, no. 1, 1 January 2018 (2018-01-01), pages 65 - 70, XP055936026, ISSN: 2213-1779, DOI: 10.1016/j.jchf.2017.10.001

## Description

### CROSS-REFERENCE TO RELATED APPLICATION

This application claims the benefit of, and priority to, U.S. Patent Application No. 63/169,998, filed on April 2, 2021.

### BACKGROUND

Furosemide, an exemplary loop diuretic, can be used in the treatment of hypertension, edema and related conditions, including decompensated heart failure. Furosemide is commonly used in the treatment and/or management of edema associated with cardiac, renal, and hepatic insufficiency or failure, for example, congestive heart failure. H. Bundgaard, T. Norgaard. N. M. Nielsen, "Photodegradation and hydrolysis of furosemide and furosemide esters in aqueous solutions," International Journal of Pharmaceutics 42, 217 (1988).

Oral bioavailability, and therefore oral efficacy, of furosemide is limited. Furosemide is commonly administered both parenterally and orally, although highly variable oral absorption is observed due to the combined effects of limited solubility and decreased stability at acidic pH. B. Devarakonda. D. P. Otto, A. Judefeind, R. A. Hill, M. M. de Villiers, "Effect of pH on the solubility and release of furosemide from polyamidoamine (PAMAM) dendrimer complexes," International Journal of Pharmaceutics 345, 142 (Dec. 10, 2007). Accordingly, furosemide typically is administered intravenously or intramuscularly for most patients with decompensated heart failure or other forms of more advanced edema.

Intravenous administration of a pharmaceutical drug, such as furosemide, requires a trained healthcare professional for placement of the catheter and administration of the drug solution. In contrast, subcutaneous administration of a pharmaceutical drug can be accomplished with the aid of auto-injection devices and/or minipumps or subcutaneous injections or infusions, which can permit administration to be performed by the patient or caregiver, for example, at home.

For subcutaneous administration, discomfort and pain during administration should be minimized so as to avoid poor patient compliance with the treatment regimen. Factors that can contribute to pain and discomfort perceived by a patient upon, during, or after subcutaneous administration include the injection volume, the pH of the formulation, and the osmolarity or osmolality of the formulation. Moreover, such a formulation should be stable in solution so that it readily is available for use and/or can be pre-loaded into a variety of dispensing devices. NCT04161482,ClinicalTrials.gov, 10 July 2020 (2020-07-10) discloses an open label study to evaluate the impact of duration of subcutaneous infusion of a pH neutral formulation of Furosemide (Furoscix^{®}) on Safety and local skin tolerability.

Thus, the art desires new and alternative formulations and delivery vehicles and profiles for the subcutaneous administration of furosemide at therapeutically effective amounts.

### SUMMARY

Note that the references to the methods of treatment by therapy of this description are to be interpreted as references to compounds, pharmaceutical compositions and medicaments of the present invention for use in those methods.

In one aspect, the invention provides methods of treating a condition selected from the group consisting of congestion due to fluid overload, edema, and hypertension, in a human in need thereof, wherein the methods generally comprise administering a liquid pharmaceutical formulation comprising furosemide. In certain embodiments, a therapeutically effective amount of furosemide is subcutaneously administered, for example, over a period of about 0.5 hours to about 2 hours. That is, it has been discovered that liquid pharmaceutical formulations of the invention. 10 mL in total volume, including a therapeutically effective amount of furosemide, 80 mg, can be delivered subcutaneously over reduced time periods, such as from about 30 minutes (0.5 hour), about one hour, about 1.5 hours, about 2 hours Delivery of 80 mg furosemide in a volume of 10 mL during reduced time periods without substantial increase in pain at the site of injection can permit a patient to wear an infusion device or the like for shorter periods of time, which should increase compliance with the treatment.

In various embodiments of the invention, the method comprises administering subcutaneously to the human a total volume of 10 mL of a liquid pharmaceutical formulation comprising 80 mg furosemide over a period of about 0.5 hours to about 2 hours.

In certain embodiments, the method, about 2 hours after administration is initiated, results in a total urine output of about 450 mL to about 950 mL. In certain embodiments, the method, about 6 hours after administration is initiated, results in a total urine output of about 2100 mL to about 2600 mL. In certain embodiments, the method, about 24 hours after administration is initiated, results in a total urine output of about 3200 mL to about 4200 mL. In certain embodiments, the method, about 2 hours after administration is initiated, results in a total urine sodium excretion of about 40 mmol to about 110 mmol. In certain embodiments, the method, about 6 hours after administration is initiated, results in a total urine sodium excretion of about 220 mmol to about 300 mmol. In certain embodiments, the method, about 24 hours after administration is initiated, results in a total urine sodium excretion of about 350 mmol to about 480 mmol.

In various embodiments of the invention, the method comprises administering subcutaneously to the human a liquid pharmaceutical formulation comprising furosemide over a period of about 0.5 hours to about 2 hours, wherein one or more of the following:
the method, about 2 hours after administration is initiated, results in a total urine output of about 450 mL to about 950 mL;
the method, about 6 hours after administration is initiated, results in a total urine output of about 2100 mL to about 2600 mL;
the method, about 24 hours after administration is initiated, results in a total urine output of about 3200 mL to about 4200 mL;
the method, about 2 hours after administration is initiated, results in a total urine sodium excretion of is about 40 mmol to about 110 mmol;
the method, about 6 hours after administration is initiated, results in a total urine sodium excretion of about 220 mmol to about 300 mmol; and
the method, about 24 hours after administration is initiated, results in a total urine sodium excretion of about 350 mmol to about 480 mmol.

In certain embodiments, the human is administered a total volume of 10 mL of the liquid pharmaceutical formulation. In certain embodiments, the liquid pharmaceutical formulation comprises 80 mg furosemide

In various embodiments of the invention, the method comprises administering subcutaneously to the human a total volume of 10 mL of a liquid pharmaceutical formulation comprising 80 mg furosemide over a period about 0.5 hours to about 2 hours, wherein one or more of the following:
the method, about 2 hours after administration is initiated, results in a total urine output of about 2100 mL to about 2600 mL;
the method, about 6 hours after administration is initiated, results in a total urine output of about 2100 mL to about 2600 mL;
the method, about 24 hours after administration is initiated, results in a total urine output of about 3200 mL to about 4200 mL;
the method, about 2 hours after administration is initiated, results in a total urine sodium excretion of about 40 mmol to about 110 mmol;
the method, about 6 hours after administration is initiated, results in a total urine sodium excretion of about 220 mmol to about 300 mmol; and
the method, about 24 hours after administration is initiated, results in a total urine sodium excretion of about 350 mmol to about 480 mmol.

Administering is over a period of about 0.5 hours to about 2 hours. In certain embodiments, administering comprises a continuous delivery profile over a period of about two hours. In certain embodiments, administering comprises a bi-phasic delivery profile over a period of about two hours. In some embodiments, the bi-phasic delivery profile results in the delivery of about 40 mg furosemide over about the first 0.5 hour of the period of two hours and about 40 mg furosemide over about the last 0.5 hour of the period of two hours. In certain embodiments, administering comprises a continuous delivery profile over a period of about 1.5 hours. In certain embodiments, administering comprises a continuous delivery profile over a period of about one hour. In some embodiments, administering comprises a continuous delivery profile over a period of about 0.5 hours.

In certain embodiments, the liquid pharmaceutical formulation further comprises a pharmaceutically acceptable buffer. In some embodiments, the pharmaceutically acceptable buffer comprises tromethamine or a pharmaceutically acceptable salt thereof. In some embodiments, the buffering agent is tromethamine hydrochloride. In certain embodiments, the liquid pharmaceutical formulation further comprises about 79 mg tromethamine hydrochloride, and water. In certain embodiments, the liquid pharmaceutical formulation has a pH of about 7.2 to about 8. In certain embodiments, the liquid pharmaceutical composition further comprises one or more pharmaceutically acceptable excipients, such as benzyl alcohol, sodium chloride, sodium hydroxide, hydrochloric acid, and combinations thereof. In certain embodiments, the liquid pharmaceutical formulation is isosmotic.

In certain embodiments, the liquid pharmaceutical formulation is administered from a prefilled polymeric cartridge. In certain embodiments, the polymeric prefilled cartridge is associated with an on-body wearable delivery device. In certain embodiments, the polymeric cartridge of the polymeric prefilled cartridge comprises a cyclic olefin polymer. In certain embodiments, the polymeric prefilled cartridge is a single-use cartridge.

In certain embodiments, the method, during administrating of the liquid pharmaceutical formulation, results in a pain score of 0, wherein the pain score is measured using an 11-point numeric pain assessment scale. In certain embodiments, the method, nearly immediately after administration of the liquid pharmaceutical formulation is complete, results in a pain score of 0, wherein the pain score is measured using an 11-point numeric pain assessment scale.

In certain embodiments, the human is an adult human. In certain embodiments, the adult human has New York Heart Association (NYHA) Class II, Class III or Class IV heart failure, liver failure, or renal failure. In certain embodiments, the adult human displays reduced responsiveness to oral diuretics before beginning subcutaneous administration according to the method. In certain embodiments, the adult human is 45 to 80 years of age.

In certain embodiments, the edema is associated with congestive heart failure, cirrhosis of the liver or renal disease. In some embodiments, the renal disease is nephrotic syndrome.

In another aspect, the invention provides a liquid pharmaceutical formulation comprising furosemide for use in a method of treating a condition selected from the group consisting of congestion due to fluid overload, edema, and hypertension, in a human in need thereof.

In various embodiments, the invention provides a liquid pharmaceutical formulation comprising 80 mg furosemide for use in a method of treating a condition selected from the group consisting of congestion due to fluid overload, edema, and hypertension, in a human in need thereof; wherein the liquid pharmaceutical formulation is subcutaneously administered to the human over a period of about 0.5 hours to about 2 hours.

In some embodiments, the invention provides a liquid pharmaceutical formulation comprising 80 mg furosemide for use in a method of treating a condition selected from the group consisting of congestion due to fluid overload, edema, and hypertension, in a human in need thereof; wherein the liquid pharmaceutical formulation is subcutaneously administered to the human over a period of about 0.5 hours to about 2 hours; and wherein
the method, about 2 hours after administration is initiated, results in a total urine output of about 450 mL to about 950 mL;
the method, about 6 hours after administration is initiated, results in a total urine output of about 2100 mL to about 2600 mL;
the method, about 24 hours after administration is initiated, results in a total urine output of about 3200 mL to about 4200 mL;
the method, about 2 hours after administration is initiated, results in a total urine sodium excretion of is about 40 mmol to about 110 mmol;
the method, about 6 hours after administration is initiated, results in a total urine sodium excretion of about 220 mmol to about 300 mmol; and
the method, about 24 hours after administration is initiated, results in a total urine sodium excretion of about 350 mmol to about 480 mmol.

### BRIEF DESCRIPTION OF THE DRAWINGS

FIG. 1 is a flow chart summarizing tasks 1-5 of the furosemide pharmacokinetic and pharmacodynamic modeling study described in Example 2.
FIG. 2 is a plot depicting the urine volume rate of excretion vs time profile expressed as mL/hr at each mid-point of the time duration (e.g.. 0.5. 1, 3, 5, 7. 9, 11, 15 and 21 hr) for subcutaneous (SC) and intravenous (IV) administration of furosemide, as further described in Example 2.
FIG. 3 is a plot depicting the observed vs simulated cumulative urine volume output following administration of SC furosemide treatment (furosemide administered at 30 mg over the first hour and then at 12.5 mg per hour over the subsequent 4 hours), as further described in Example 2.
FIG. 4 is a plot depicting total urine volume output as a function of time for SC and IV administration of furosemide (40 mg furosemide subcutaneously administered at 2 timepoints, time 0 and 1.5 hr (over 30 minutes each time)), as further described in Example 2.
FIG. 5 is a plot depicting total urine volume output as a function of time for SC and IV administration of furosemide (80 mg furosemide subcutaneously administered over 1 hour), as further described in Example 2.
FIG. 6 is a plot depicting total urine volume output as a function of time for SC and IV administration of furosemide (80 mg furosemide subcutaneously administered over 2 hours), as further described in Example 2.
FIG. 7 is a plot depicting total urine volume output as a function of time for SC and IV administration of furosemide (80 mg furosemide subcutaneously administered over 4 hours), as further described in Example 2.
FIG. 8 is a plot depicting total urine volume output as a function of time for SC and IV administration of furosemide (80 mg furosemide subcutaneously administered over 8 hours), as further described in Example 2.

### DETAILED DESCRIPTION

It has now been discovered that a therapeutically effective amount of furosemide in a volume of about 10 mL can be delivered subcutaneously during reduced time periods without substantial increase in pain at the site of injection. Such methods can permit a patient to wear an infusion device or the like for shorter periods of time, which should increase compliance with the treatment. As generally described herein, the invention provides methods of treating various conditions, diseases, and disorders (e.g., congestion due to fluid overload, edema, and hypertension) in a patient (human) in need thereof. The methods generally can comprise subcutaneously administering a liquid pharmaceutical formulation of furosemide 80 mg) to the patient over a period of about 0.5 hours to about 2 hours). The liquid pharmaceutical formulations of furosemide and delivery profiles described herein can result in optimized furosemide pharmacokinetic and pharmacodynamic profiles that can maximize patient outcomes (e.g., total urine output and urine sodium excretion).

### Definitions

To facilitate an understanding of the present invention, a number of terms and phrases are defined below.

Unless defined otherwise, all technical and scientific terms used herein have the same meaning as commonly understood by one of ordinary skill in the art to which this invention belongs. The abbreviations used herein have their conventional meaning within the chemical and biological arts. The chemical structures and formulae set forth herein are constructed according to the standard rules of chemical valency known in the chemical arts.

The terms "a" and "an" as used herein mean "one or more" and include the plural unless the context is inappropriate.

In the application, where an element or component is said to be included in and/or selected from a list of recited elements or components, it should be understood that the element or component can be any one of the recited elements or components, or the element or component can be selected from a group consisting of two or more of the recited elements or components.

Further, it should be understood that elements and/or features of a composition or a method described herein can be combined in a variety of ways without departing from the spirit and scope of the present invention, whether explicit or implicit herein. For example, where reference is made to a particular compound, that compound can be used in various embodiments of compositions of the present invention and/or in methods of the present invention, unless otherwise understood from the context. In other words, within this application, embodiments have been described and depicted in a way that enables a clear and concise application to be written and drawn, but it is intended and will be appreciated that embodiments may be variously combined or separated without parting from the present teachings and invention(s). For example, it will be appreciated that all features described and depicted herein can be applicable to all aspects of the invention(s) described and depicted herein.

It should be understood that the expression "at least one of" includes individually each of the recited objects after the expression and the various combinations of two or more of the recited objects unless otherwise understood from the context and use. The expression "and/or" in connection with three or more recited objects should be understood to have the same meaning unless otherwise understood from the context.

The use of the tenn "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

Where the use of the term "about" is before a quantitative value, the present invention also includes the specific quantitative value itself, unless specifically stated otherwise. As used herein, the term "about" refers to a ±10% variation from the nominal value unless otherwise indicated or inferred from the context.

At various places in the present specification, values are disclosed in groups or in ranges. It is specifically intended that the description include each and every individual subcombination of the members of such groups and ranges. For example, an integer in the range of 0 to 40 is specifically intended to individually disclose 0, 1, 2, 3, 4, 5, 6, 7, 8. 9, 10, 11, 12, 13, 14, 15. 16, 17, 18, 19, 20, 21, 22, 23, 24, 25, 26, 27, 28, 29, 30, 31, 32, 33, 34, 35, 36, 37, 38, 39, and 40, and an integer in the range of 1 to 20 is specifically intended to individually disclose 1, 2, 3, 4, 5, 6, 7, 8, 9, 10, 11, 12, 13, 14. 15, 16, 17, 18, 19, and 20.

The use of any and all examples, or exemplary language herein, for example, "such as" or "including," is intended merely to illustrate better the present invention and does not pose a limitation on the scope of the invention unless claimed. No language in the specification should be construed as indicating any non-claimed element as essential to the practice of the present invention.

As used herein, a "compound" (including a specifically named compound. e.g.. furosemide) refers to the compound itself and its pharmaceutically acceptable salts unless otherwise understood from the context of the description or expressly limited to one particular form of the compound, e.g., the compound itself, or a pharmaceutically acceptable salt thereof.

As used herein. "furosemide" refers to a compound having the formula: 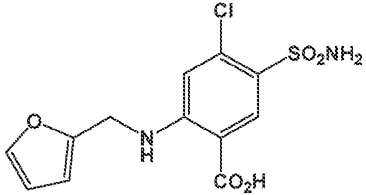 and pharmaceutically acceptable salts thereof. Such salts may include, but are not limited to, furosemide sodium salt and furosemide quaternary ammonium salt. Furosemide may be referred to by other names, for example, frusemide, 5-(aminosulphonyl)-4-chloro-2-[(2-furanyl-methyl)amino]benzoic acid, or its IUPAC name, 4-chloro-2-(furan-2-ylmethylamino)-5-sulfamoyl-benzoic acid, or its common trade name, Lasix^{®}.

As used herein, the terms "subject" and "patient" refer to organisms to be treated by the methods and/or compositions described herein. Such organisms are preferably mammals (e.g., murines, simians, equines, bovines, porcines, canines, felines, and the like), and more preferably humans.

As used herein, a "buffer" refers to an aqueous solution that is resistant to changes in pH. A buffer may include a "buffering agent" such as a weak acid and its salt, or a weak base and its salt, which assist in maintaining the stability of the pH. Examples of buffers used in pharmaceutical formulations include bicarbonate buffers, carbonate buffers, citrate buffers, histidine buffers, phosphate buffers, tartrate buffers, tris(hydroxymethyl)aminomethane (or 2-amino-2-hydroxymethyl-propane-1,3-diol [(HOCH₂)₃CNH₂]) buffers, and combinations thereof. Certain of these buffers are suitable for pharmaceutical formulations administered subcutaneously.

Tris(hydroxymethyl)aminomethane or a tris(hydroxymethyl)aminomethane buffer can be referred to as "TRIS," "Tris," "Tris buffer," "Trisamine," "THAM," "tromethamine," and other names. In addition, many buffers and/or buffer systems can include Tris, or a pharmaceutically acceptable salt thereof, and can be used in the present teachings. For example, Tris-buffered saline ("TBS"). Tris-hydrochloride buffer ("Tris-HCl"), Tris base (pH 10.6), Tris/borate/ethylene diamine tetra-acetate ("EDTA") buffer (''TBE"), and Tris/acetate/EDTA buffer ("TAE"). Tris base often is used with Tris-HCl to prepare Tris buffers at a desired pH. In addition, the present teachings can include Tris-related compounds, for example, compounds derived from Tris or structurally-related to Tris, that can act as a buffer.

As used herein, "tonicity" refers to the ionic strength or concentration of ions in a solution such as a pharmaceutical formulation. Tonicity often is measured in molarity ("M"). As used herein, an "isotonic solution." an "isotonic formulation," an "isotonic pharmaceutical formulation," and a pharmaceutical formulation that is "isotonic" refers to a solution or formulation that has the same or similar concentration of ions as found in bodily fluids.

As used herein, "physiological pH" refers to a pH of about 7.4.

As used herein, "osmoticity" and "osmolality" refer to the osmotic pressure of a solution such as a pharmaceutical formulation. Osmoticity often is measured in osmolarity ("Osm/L" or "OsM") or osmolality ("Osm/kg"), which can be used interchangeably herein. When measuring freezing point depression, the observed value is the osmolality of the solution. In contrast to tonicity, osmoticity accounts for un-ionized solutes in a solution such that when present, the osmolarity or osmolality of the solution will be higher than its tonicity. The osmolarity of a liquid pharmaceutical formulation described herein can be measured, for example, using a vapor pressure method.

As used herein, an "isosmotic solution." an "isosmotic formulation," an "isosmotic pharmaceutical formulation," and a pharmaceutical formulation that is "isosmotic" refers to a solution or a formulation that has the same or similar concentration of solutes as found in bodily fluids. In certain embodiments, a liquid pharmaceutical formulation that is "isosmotic" can have an osmolarity in the range of about 275 mOsM to about 350 mOsM or when the osmolality of the formulation is in the range of about 275 mOsm/kg to about 350 mOsm/kg.

As used herein, "osmolarity adjustor" and "osmotic agent" refer to a pharmaceutically acceptable compound that may be added to a liquid pharmaceutical formulation described herein in order to modulate the osmolarity of the liquid pharmaceutical formulation.

As used herein. "pharmaceutically acceptable" refers to a substance that is acceptable for use in pharmaceutical applications from a toxicological perspective and does not adversely interact with the active ingredient. Accordingly, pharmaceutically acceptable carriers are those that are compatible with the other ingredients in the formulation and are biologically acceptable. In certain embodiments, supplementary active ingredients can also be incorporated into the pharmaceutical compositions.

As used herein. "pharmaceutically acceptable excipient" refers to a substance that aids the administration of an active agent to and absorption by a subject and can be included in the compositions of the present invention without causing a significant adverse toxicological effect on the patient. Non-limiting examples of pharmaceutically acceptable excipients include water, NaCl, normal saline solutions, a phosphate buffered saline solution, emulsions (e.g.. such as an oil/water or water/oil emulsions), lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxypropylmethylcellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. For examples of excipients and carriers, *see* Martin, Remington's Pharmaceutical Sciences, 15th Ed., Mack Publ. Co., Easton, PA (1975).

As used herein, the term "pharmaceutically acceptable carrier" refers to any of the standard pharmaceutical carriers, such as a phosphate buffered saline solution, water, emulsions (e.g., such as an oil/water or water/oil emulsions), and various types of wetting agents. The compositions also can include stabilizers and preservatives. For examples of carriers, stabilizers and adjuvants, see Martin, Remington's Pharmaceutical Sciences, 15th Ed., Mack Publ. Co., Easton, PA (1975).

As used herein, the term "pharmaceutically acceptable salt" refers to any pharmaceutically acceptable salt (*e.g.,* acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metal (e.g., sodium) hydroxides, alkaline earth metal (e.g.. magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

As used herein, the term "effective amount" refers to the amount of a composition (e.g.. a liquid pharmaceutical formulation of the present invention) sufficient to effect beneficial or desired results. An effective amount can be administered in one or more administrations, applications or dosages and is not intended to be limited to a particular formulation or administration route.

As used herein, the terms "treat," "treating," and "treatment" include any effect, e.g., lessening, reducing, modulating, ameliorating or eliminating, that results in the improvement of the condition, disease, disorder, and the like, or ameliorating a symptom thereof.

The phrase "therapeutically-effective amount" as used herein means that amount of a composition (e.g., a liquid pharmaceutical formulation of the present invention) which is effective for producing some desired therapeutic effect in a subject.

As used herein, the term "congestion" (in heart failure) is the presence of signs and symptoms of extracellular fluid accumulation that results in increased cardiac filling pressures leading to reduced cardiac output. This reduced cardiac output is further exacerbated by neurohormonal activation leading to increased renal sodium and water avidity resulting in an increased plasma volume.

As used herein, "fluid overload," "volume overload" and "hypervolemia", may describe a medical condition where there is too much fluid in the blood. Excess fluid, primarily salt and water, may build up throughout the body resulting in weight gain.

Throughout the description, where compositions and kits are described as having, including, or comprising specific components, or where processes and methods are described as having, including, or comprising specific steps, it is contemplated that, additionally, there are compositions and kits of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### Liquid Pharmaceutical Formulations of Furosemide

As described herein, in one aspect, the invention provides liquid pharmaceutical formulations comprising furosemide, or a pharmaceutically acceptable salt thereof, for the treatment of a condition described herein. In various embodiments, the condition is selected from the group consisting of congestion due to fluid overload, edema, and hypertension, and combinations thereof. In certain embodiments, the condition is congestion due to fluid overload. In certain embodiments, the condition is edema. In certain embodiments, the condition is hypertension.

In various embodiments, the liquid pharmaceutical formulations described herein can comprise about 60 mg to about 100 mg, about 65 mg to about 100 mg, about 70 mg to about 100 mg, about 75 mg to about 100 mg, about 80 mg to about 100 mg, about 85 mg to about 100 mg, about 90 mg to about 100 mg, about 95 mg to about 100 mg, about 60 mg to about 95 mg, about 60 mg to about 90 mg, about 60 mg to about 85 mg, about 60 mg to about 80 mg, about 60 mg to about 75 mg, about 60 mg to about 70 mg, about 60 mg to about 65 mg, about 65 mg to about 95 mg, about 65 mg to about 90 mg, about 65 mg to about 85 mg, about 65 mg to about 80 mg, about 65 mg to about 75 mg, about 65 mg to about 70 mg, about 70 mg to about 95 mg, about 70 mg to about 90 mg, about 70 mg to about 85 mg, about 70 mg to about 80 mg, about 70 mg to about 75 mg, about 75 mg to about 95 mg, about 75 mg to about 90 mg, about 75 mg to about 85 mg, about 75 mg to about 80 mg, about 80 mg to about 95 mg, about 80 mg to about 90 mg, about 80 mg to about 85 mg, about 85 mg to about 95 mg, about 85 mg to about 90 mg, or about 90 mg to about 95 mg furosemide.

In various embodiments, the liquid pharmaceutical formulations described herein can comprise about 60 mg, about 65 mg, about 70 mg, about 75 mg, about 80 mg, about 85 mg, about 90 mg, about 95 mg, or about 100 mg furosemide. According to the present invention, the liquid pharmaceutical formulations comprise 80 mg furosemide.

In various embodiments, the liquid pharmaceutical formulations described herein can comprise a pharmaceutically acceptable salt of furosemide.

In various embodiments, a pharmaceutically acceptable salt of furosemide described herein can be present in the liquid pharmaceutical formulations in an amount that provides about 60 mg to about 100 mg, about 65 mg to about 100 mg, about 70 mg to about 100 mg, about 75 mg to about 100 mg, about 80 mg to about 100 mg, about 85 mg to about 100 mg, about 90 mg to about 100 mg, about 95 mg to about 100 mg, about 60 mg to about 95 mg, about 60 mg to about 90 mg, about 60 mg to about 85 mg, about 60 mg to about 80 mg, about 60 mg to about 75 mg, about 60 mg to about 70 mg, about 60 mg to about 65 mg, about 65 mg to about 95 mg, about 65 mg to about 90 mg, about 65 mg to about 85 mg, about 65 mg to about 80 mg, about 65 mg to about 75 mg, about 65 mg to about 70 mg, about 70 mg to about 95 mg, about 70 mg to about 90 mg, about 70 mg to about 85 mg, about 70 mg to about 80 mg, about 70 mg to about 75 mg, about 75 mg to about 95 mg, about 75 mg to about 90 mg, about 75 mg to about 85 mg, about 75 mg to about 80 mg, about 80 mg to about 95 mg, about 80 mg to about 90 mg, about 80 mg to about 85 mg, about 85 mg to about 95 mg, about 85 mg to about 90 mg, or about 90 mg to about 95 mg furosemide in its free base form.

In various embodiments, a pharmaceutically acceptable salt of furosemide described herein can be present in the liquid pharmaceutical formulations in an amount that provides about 60 mg, about 65 mg, about 70 mg, about 75 mg, about 80 mg, about 85 mg, about 90 mg, about 95 mg, or about 100 mg furosemide in its free base form. According to the invention, the pharmaceutically acceptable salt of furosemide is present in the liquid pharmaceuticals formulation in an amount that provides 80 mg furosemide in its free base form

In various embodiments, the concentration of furosemide in the liquid pharmaceutical formulations described herein can be about 5 mg/mL to about 15 mg/mL, about 6 mg/mL to about 15 mg/mL, about 7 mg/mL to about 15 mg/mL, about 8 mg/mL to about 15 mg/mL, about 9 mg/mL to about 15 mg/mL, about 10 mg/mL to about 15 mg/mL, about 11 mg/mL to about 15 mg/mL, about 12 mg/mL to about 15 mg/mL, about 13 mg/mL to about 15 mg/mL, about 14 mg/mL to about 15 mg/mL, about 5 mg/mL to about 14 mg/mL, about 5 mg/mL to about 13 mg/mL, about 5 mg/mL to about 12 mg/mL, about 5 mg/mL to about 11 mg/mL, about 5 mg/mL to about 10 mg/mL, about 5 mg/mL to about 9 mg/mL, about 5 mg/mL to about 8 mg/mL, about 5 mg/mL to about 7 mg/mL. about 5 mg/mL to about 6 mg/mL, about 6 mg/mL to about 14 mg/mL, about 6 mg/mL to about 13 mg/mL, about 6 mg/mL to about 12 mg/mL. about 6 mg/mL to about 11 mg/mL, about 6 mg/mL to about 10 mg/mL, about 6 mg/mL to about 9 mg/mL, about 6 mg/mL to about 8 mg/mL, about 6 mg/mL to about 7 mg/mL. about 7 mg/mL to about 14 mg/mL, about 7 mg/mL to about 13 mg/mL, about 7 mg/mL to about 12 mg/mL, about 7 mg/mL to about 11 mg/mL, about 7 mg/mL to about 10 mg/mL, about 7 mg/mL to about 9 mg/mL, about 7 mg/mL to about 8 mg/mL, about 8 mg/mL to about 14 mg/mL, about 8 mg/mL to about 13 mg/mL, about 8 mg/mL to about 12 mg/mL, about 8 mg/mL to about 11 mg/mL. about 8 mg/mL to about 10 mg/mL. about 8 mg/mL to about 9 mg/mL, about 9 mg/mL to about 14 mg/mL, about 9 mg/mL to about 13 mg/mL, about 9 mg/mL to about 12 mg/mL, about 9 mg/mL to about 11 mg/mL. about 9 mg/mL to about 10 mg/mL, about 10 mg/mL to about 14 mg/mL, about 10 mg/mL to about 13 mg/mL, about 10 mg/mL to about 12 mg/mL, about 10 mg/mL to about 11 mg/mL, about 11 mg/mL to about 14 mg/mL, about 11 mg/mL to about 13 mg/mL, about 11 mg/mL to about 12 mg/mL, about 12 mg/mL to about 14 mg/mL, about 12 mg/mL to about 13 mg/mL, or about 13 mg/mL to about 14 mg/mL.

In various embodiments, the concentration of furosemide in the liquid pharmaceutical formulations described herein can be about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, or about 15 mg/mL. According to the invention, the concentration of furosemide in the liquid pharmaceutical formulation is 8 mg/mL.

In various embodiments, the liquid pharmaceutical fonnulations described herein may further comprise a pharmaceutically acceptable buffer.

In various embodiments, a liquid pharmaceutical composition comprises:
(i) furosemide, or a pharmaceutically acceptable salt thereof; and
(ii) a pharmaceutically acceptable buffer.

In certain embodiments, the pharmaceutically acceptable buffer comprises a buffering agent selected from the group consisting of histidine, a citrate salt, sodium phosphate, potassium phosphate, tromethamine or a pharmaceutically acceptable salt thereof, and combinations thereof. In certain embodiments, the buffering agent is tromethamine, or a pharmaceutically acceptable salt thereof. In certain embodiments, the pharmaceutically acceptable salt of tromethamine is tromethamine hydrochloride.

In various embodiments, the liquid pharmaceutical formulations described herein may further comprise water.

In various embodiments, a liquid pharmaceutical formulation comprises:
(i) furosemide, or a pharmaceutically acceptable salt thereof;
(ii) a pharmaceutically acceptable buffer: and
(iii) water.

In various embodiments, the liquid pharmaceutical formulations described herein can comprise about 75 mg to about 85 mg, about 76 mg to about 85 mg, about 77 mg to about 85 mg, about 78 mg to about 85 mg, about 79 mg to about 85 mg, about 80 mg to about 85 mg, about 81 mg to about 85 mg, about 82 mg to about 85 mg, about 83 mg to about 85 mg, about 84 mg to about 85 mg, about 75 mg to about 84 mg, about 75 mg to about 83 mg, about 75 mg to about 82 mg, about 75 mg to about 81 mg, about 75 mg to about 80 mg, about 75 mg to about 79 mg, about 75 mg to about 78 mg, about 75 mg to about 77 mg, about 75 mg to about 76 mg, about 76 mg to about 84 mg, about 76 mg to about 83 mg, about 76 mg to about 82 mg, about 76 mg to about 81 mg, about 76 mg to about 80 mg, about 76 mg to about 79 mg, about 76 mg to about 78 mg, about 76 mg to about 77 mg, about 77 mg to about 84 mg, about 77 mg to about 83 mg, about 77 mg to about 82 mg, about 77 mg to about 81 mg, about 77 mg to about 80 mg, about 77 mg to about 79 mg, about 77 mg to about 78 mg, about 78 mg to about 84 mg, about 78 mg to about 83 mg, about 78 mg to about 82 mg, about 78 mg to about 81 mg, about 78 mg to about 80 mg, about 78 mg to about 79 mg, about 79 mg to about 84 mg, about 79 mg to about 83 mg, about 79 mg to about 82 mg, about 79 mg to about 81 mg, about 79 mg to about 80 mg, about 80 mg to about 84 mg, about 80 mg to about 83 mg, about 80 mg to about 82 mg, about 80 mg to about 81 mg, about 81 mg to about 84 mg, about 81 mg to about 83 mg, about 81 mg to about 82 mg, about 82 mg to about 84 mg, about 82 mg to about 83 mg, or about 83 mg to about 84 mg tromethamine hydrochloride.

In various embodiments, the liquid pharmaceutical formulations described herein can comprise about 75 mg, about 76 mg, about 77 mg, about 78 mg, about 79 mg, about 80 mg, about 81 mg, about 82 mg, about 83 mg, about 84 mg, or about 85 mg tromethamine hydrochloride. In certain embodiments, the liquid pharmaceutical formulations described herein comprise about 79 mg tromethamine hydrochloride.

In various embodiments, the concentration of tromethamine hydrochloride in the liquid pharmaceutical formulation is about 25 mmol to about 500 mmol, about 50 mmol to about 500 mmol, about 100 mmol to about 500 mmol, about 150 mmol to about 500 mmol, about 200 mmol to about 500 mmol, about 250 mmol to about 500 mmol, about 300 mmol to about 500 mmol, about 400 mmol to about 500 mmol, about 25 mmol to about 400 mmol, about 25 mmol to about 300 mmol, about 25 mmol to about 250 mmol, about 25 mmol to about 200 mmol, about 25 mmol to about 150 mmol, about 25 mmol to about 100 mmol, about 25 mmol to about 50 mmol, about 50 mmol to about 400 mmol, about 50 mmol to about 300 mmol, about 50 mmol to about 250 mmol, about 50 mmol to about 200 mmol, about 50 mmol to about 150 mmol, about 50 mmol to about 100 mmol, about 100 mmol to about 400 mmol, about 100 mmol to about 300 mmol, about 100 mmol to about 250 mmol, about 100 mmol to about 200 mmol, about 100 mmol to about 150 mmol, about 150 mmol to about 400 mmol, about 150 mmol to about 300 mmol, about 150 mmol to about 250 mmol, about 150 mmol to about 200 mmol, about 200 mmol to about 400 mmol, about 200 mmol to about 300 mmol, about 200 mmol to about 250 mmol, about 250 mmol to about 400 mmol, about 250 mmol to about 300 mmol, or about 300 mmol to about 400 mmol.

In various embodiments, the concentration of tromethamine hydrochloride in the liquid pharmaceutical formulation is about 25 mmol, about 50 mmol, about 100 mmol, about 150 mmol, about 200 mmol, about 250 mmol, about 300 mmol, about 400 mmol, or about 500 mmol.

In various embodiments, the molar ratio of tromethamine hydrochloride to furosemide, or a pharmaceutically acceptable salt thereof, in the liquid pharmaceutical formulation is about 1 to about 3.5, about 1.5 to about 3.5, about 2 to about 3.5, about 2.5 to about 3.5, about 3 to about 3.5, about 1 to about 3, about 1 to about 2.5, about 1 to about 2, about 1 to about 1.5, about 1.5 to about 3, about 1.5 to about 2.5, about 1.5 to about 2, about 2 to about 3, about 2 to about 2.5, or about 2.5 to about 3.

In various embodiments, the molar ratio of tromethamine hydrochloride to furosemide, or a pharmaceutically acceptable salt thereof, in the liquid pharmaceutical formulation is about 1, about 1.5, about 2, about 2.5, about 3, or about 3.5.

In various embodiments, the molar ratio of tromethamine hydrochloride to furosemide in the liquid pharmaceutical formulation is about 1 to about 3.5, about 1.5 to about 3.5, about 2 to about 3.5, about 2.5 to about 3.5, about 3 to about 3.5, about 1 to about 3, about 1 to about 2.5, about 1 to about 2, about 1 to about 1.5, about 1.5 to about 3, about 1.5 to about 2.5, about 1.5 to about 2, about 2 to about 3, about 2 to about 2.5, or about 2.5 to about 3.

In various embodiments, the molar ratio of tromethamine hydrochloride to furosemide in the liquid pharmaceutical formulation is about 1, about 1.5, about 2, about 2.5, about 3, or about 3.5.

In various embodiments, the liquid pharmaceutical compositions described herein can comprise about 8 mL to about 12 mL. about 8.5 mL to about 12 mL, about 9 mL to about 12 mL, about 9.5 mL to about 12 mL, about 10 mL to about 12 mL, about 10.5 mL to about 12 mL, about 11 mL to about 12 mL, about 11.5 mL to about 12 mL, about 8 mL to about 11.5 mL, about 8 mL to about 11 mL, about 8 mL to about 10.5 mL, about 8 mL to about 10 mL, about 8 mL to about 9.5 mL, about 8 mL to about 9 mL, about 8 mL to about 8.5 mL, about 8.5 mL to about 11.5 mL, about 8.5 mL to about 11 mL, about 8.5 mL to about 10.5 mL, about 8.5 mL to about 10 mL, about 8.5 mL to about 9.5 mL, about 8.5 mL to about 9 mL, about 9 mL to about 11.5 mL, about 9 mL to about 11 mL, about 9 mL to about 10.5 mL, about 9 mL to about 10 mL, about 9 mL to about 9.5 mL. about 9.5 mL to about 11.5 mL, about 9.5 mL to about 11 mL, about 9.5 mL to about 10.5 mL, about 9.5 mL to about 10 mL. about 10 mL to about 11.5 mL, about 10 mL to about 11 mL, about 10 mL to about 10.5 mL, about 10.5 mL to about 11.5 mL, about 10.5 mL to about 11 mL, or about 11 mL to about 11.5 mL water.

In various embodiments, the liquid pharmaceutical compositions described herein can comprise about 8 mL, about 8.5 mL, about 9 mL, about 9.5 mL, about 10 mL, about 10.5 mL, about 11 mL, about 11.5 mL, or about 12 mL water. In certain embodiments, the liquid pharmaceutical compositions described herein can comprise about 10 mL water.

It should be understood that the volume of water added to the pharmaceutical formulation can be an amount to bring the total volume to one of the enumerated volumes of water herein, and according to the present invention, to bring the total volume of the pharmaceutical formulation to 10 mL.

In various embodiments, the liquid pharmaceutical compositions described herein can comprise about 8 g to about 12 g, about 8.5 g to about 12 g, about 9 g to about 12 g, about 9.5 g to about 12 g, about 10 g to about 12 g, about 10.5 g to about 12 g, about 11 g to about 12 g, about 11.5 g to about 12 g, about 8 g to about 11.5 g, about 8 g to about 11 g, about 8 g to about 10.5 g, about 8 g to about 10 g, about 8 g to about 9.5 g, about 8 g to about 9 g, about 8 g to about 8.5 g, about 8.5 g to about 11.5 g, about 8.5 g to about 11 g, about 8.5 g to about 10.5 g, about 8.5 g to about 10 g, about 8.5 g to about 9.5 g, about 8.5 g to about 9 g, about 9 g to about 11.5 g, about 9 g to about 11 g, about 9 g to about 10.5 g, about 9 g to about 10 g, about 9 g to about 9.5 g, about 9.5 g to about 11.5 g, about 9.5 g to about 11 g, about 9.5 g to about 10.5 g, about 9.5 g to about 10 g, about 10 g to about 11.5 g, about 10 g to about 11 g, about 10 g to about 10.5 g, about 10.5 g to about 11.5 g. about 10.5 g to about 11 g, or about 11 g to about 11.5 g water.

In various embodiments, the liquid pharmaceutical compositions described herein can comprise about 8 g. about 8.5 g, about 9 g, about 9.5 g, about 10 g, about 10.5 g, about 11 g, about 11.5 g, or about 12 g water. In certain embodiments, the liquid pharmaceutical compositions described herein can comprise about 10 g water.

In various embodiments, the liquid pharmaceutical formulations described herein may further comprise one or more pharmaceutically acceptable excipients.

In various embodiments, a liquid pharmaceutical formulation comprises:
(i) furosemide, or a pharmaceutically acceptable salt thereof;
(ii) a pharmaceutically acceptable buffer; and
(iii) one or more pharmaceutically acceptable excipients.

In various embodiments, a liquid pharmaceutical formulation comprises:
(i) furosemide, or a pharmaceutically acceptable salt thereof;
(ii) a pharmaceutically acceptable buffer;
(iii) one or more pharmaceutically acceptable excipients; and
(iv) water.

In various embodiments, the one or more pharmaceutically acceptable excipients is selected from the group consisting of ethanol, benzyl alcohol, glycerin. N-methyl-pyrrolidone (NMP), sodium chloride, sodium hydroxide, hydrochloric acid, a polyethylene glycol (PEG), propylene glycol, a polysorbate, a polyvinylpyrrolidone (PVP), a cyclodextrin, and combinations thereof. In certain embodiments, the one or more pharmaceutically acceptable excipients is selected from the group consisting of sodium chloride, sodium hydroxide, hydrochloric acid, or combinations thereof. In certain embodiments, the liquid pharmaceutical formulations described herein further comprise sodium chloride, sodium hydroxide, hydrochloric acid, or combinations thereof.

In various embodiments, the liquid pharmaceutical formulations described herein have a pH of about 6.5 to about 8.5, about 7 to about 8.5, about 7.5 to about 8.5, about 8 to about 8.5, about 6.5 to about 8, about 6.5 to about 7.5. about 6.5 to about 7, about 7 to about 8, about 7 to about 7.5, or about 7.5 to about 8. In certain embodiments, the liquid phamtaceutical formulations described herein have a pH of about 7 to about 8. In some embodiments, the liquid pharmaceutical formulations described herein have a pH of about 7.2 to about 8. In particular embodiments, the liquid pharmaceutical formulations described herein have a pH of about 7.4 to about 8.

In various embodiments, the liquid pharmaceutical formulations described herein have a pH of about 7 to about 8, about 7.2 to about 8, about 7.4 to about 8, about 7.6 to about 8, about 7.8 to about 8, about 7 to about 7.8, about 7 to about 7.6, about 7 to about 7.4, about 7 to about 7.2, about 7.2 to about 7.8, about 7.2 to about 7.6, about 7.2 to about 7.4, about 7.4 to about 7.8, about 7.4 to about 7.6. or about 7.6 to about 7.8. In certain embodiments, the liquid pharmaceutical formulations described herein have a pH of about 7 to about 7.8.

In various embodiments, the liquid pharmaceutical formulations described herein have a pH of about 6.5, about 7, about 7.5, about 8, or about 8.5.

In various embodiments, the liquid pharmaceutical formulations described herein have a pH of about 7, about 7.1, about 7.2, about 7.3, about 7.4, about 7.5. about 7.6, about 7.7. about 7.8, about 7.9, or about 8.

In various embodiments, a liquid pharmaceutical formulation comprises:
(i) furosemide, or a pharmaceutically acceptable salt thereof; and
(ii) a pharmaceutically acceptable buffer,
wherein the liquid pharmaceutical formulation has a pH of about 7 to about 7.8.

In various embodiments, a liquid pharmaceutical formulation comprises:
(i) furosemide, or a pharmaceutically acceptable salt thereof;
(ii) a pharmaceutically acceptable buffer; and
(iii) one or more pharmaceutically acceptable excipients,
wherein the liquid pharmaceutical formulation has a pH of about 7 to about 7.8.

In various embodiments, the total volume of the liquid pharmaceutical formulations described herein can be about 8 mL to about 12 mL, about 8.5 mL to about 12 mL, about 9 mL to about 12 mL, about 9.5 mL to about 12 mL, about 10 mL to about 12 mL, about 10.5 mL to about 12 mL, about 11 mL to about 12 mL, about 11.5 mL to about 12 mL, about 8 mL to about 11.5 mL, about 8 mL to about 11 mL, about 8 mL to about 10.5 mL, about 8 mL to about 10 mL, about 8 mL to about 9.5 mL, about 8 mL to about 9 mL, about 8 mL to about 8.5 mL, about 8.5 mL to about 11.5 mL, about 8.5 mL to about 11 mL, about 8.5 mL to about 10.5 mL. about 8.5 mL to about 10 mL, about 8.5 mL to about 9.5 mL, about 8.5 mL to about 9 mL, about 9 mL to about 11.5 mL, about 9 mL to about 11 mL. about 9 mL to about 10.5 mL, about 9 mL to about 10 mL, about 9 mL to about 9.5 mL, about 9.5 mL to about 11.5 mL, about 9.5 mL to about 11 mL, about 9.5 mL to about 10.5 mL, about 9.5 mL to about 10 mL, about 10 mL to about 11.5 mL, about 10 mL to about 11 mL, about 10 mL to about 10.5 mL, about 10.5 mL to about 11.5 mL, about 10.5 mL to about 11 mL, or about 11 mL to about 11.5 mL.

In various embodiments, the total volume of the liquid pharmaceutical formulations described herein can be about 8 mL, about 8.5 mL, about 9 mL, about 9.5 mL, about 10 mL, about 10.5 mL, about 11 mL, about 11.5 mL, or about 12 mL. According to the invention, the total volume of the liquid pharmaceutical formulation is 10 mL.

The liquid pharmaceutical formulation comprises 80 mg furosemide. The liquid pharmaceutical formulation comprises 80 mg furosemide and a total volume of 10 mL.

In various embodiments, a liquid pharmaceutical formulation comprises:
(i) 80 mg furosemide; and
(ii) about 79 mg tromethamine hydrochloride; and
(iii) water.

In various embodiments, a liquid pharmaceutical formulation comprises:
(i) 80 mg furosemide; and
(ii) about 79 mg tromethamine hydrochloride: and
(iii) water,
wherein the liquid pharmaceutical formulation has a total volume of about 10 mL.

In various embodiments, a liquid pharmaceutical formulation comprises:
(i) 80 mg furosemide:
(ii) about 79 mg tromethamine hydrochloride;
(iii) water; and
(iv) one or more pharmaceutically acceptable excipients.

In various embodiments, a liquid pharmaceutical formulation comprises:
(i) 80 mg furosemide:
(ii) about 79 mg tromethamine hydrochloride;
(iii) water: and
(iv) one or more pharmaceutically acceptable excipients,
wherein the liquid pharmaceutical formulation has a total volume of about 10 mL.

In various embodiments, a liquid pharmaceutical formulation comprises:
(i) 80 mg furosemide:
(ii) about 79 mg tromethamine hydrochloride;
(iii) water; and
(iv) one or more pharmaceutically acceptable excipients,
wherein the liquid pharmaceutical formulation has a pH of about 7 to about 7.8.

In various embodiments, a liquid pharmaceutical formulation comprises:
(i) 80 mg furosemide:
(ii) about 79 mg tromethamine hydrochloride:
(iii) water: and
(iv) one or more pharmaceutically acceptable excipients,
wherein the liquid pharmaceutical formulation has a pH of about 7 to about 7.8 and a total volume of 10 mL.

In various embodiments, a liquid pharmaceutical formulation comprises:
(i) 80 mg furosemide:
(ii) about 79 mg tromethamine hydrochloride:
(iii) water; and
(iv) one or more pharmaceutically acceptable excipients selected from the group consisting of sodium chloride, sodium hydroxide, hydrochloric acid, and combinations thereof.

In various embodiments, a liquid pharmaceutical formulation comprises:
(i) 80 mg furosemide:
(ii) about 79 mg tromethamine hydrochloride:
(iii) water; and
(iv) one or more pharmaceutically acceptable excipients selected from the group consisting of sodium chloride, sodium hydroxide, hydrochloric acid, and combinations thereof, wherein the liquid pharmaceutical formulation has a total volume of 10 mL

In various embodiments, a liquid pharmaceutical formulation comprises:
(i) 80 mg furosemide:
(ii) about 79 mg tromethamine hydrochloride:
(iii) water; and
(iv) one or more pharmaceutically acceptable excipients selected from the group consisting of sodium chloride, sodium hydroxide, hydrochloric acid, and combinations thereof, wherein the liquid pharmaceutical formulation has a pH of about 7 to about 7.8.

In various embodiments, a liquid pharmaceutical formulation comprises:
(i) 80 mg furosemide:
(ii) about 79 mg tromethamine hydrochloride:
(iii) water; and
(iv) one or more pharmaceutically acceptable excipients selected from the group consisting of sodium chloride, sodium hydroxide, hydrochloric acid, and combinations thereof,
wherein the liquid pharmaceutical formulation has a pH of about 7 to about 7.8 and a total volume of 10 mL.

In various embodiments, the liquid pharmaceutical formulations described herein have an osmolarity in the range of about 250 mOsM (or 250 mOsm/kg) to about 350 mOsM (or 350 mOsm/kg), about 275 mOsM (or 275 mOsm/kg) to about 350 mOsM (or 350 mOsm/kg), about 300 mOsM (or 300 mOsm/kg) to about 350 mOsM (or 350 mOsm/kg), about 325 mOsM (or 325 mOsm/kg) to about 350 mOsM (or 350 mOsm/kg), about 250 mOsM (or 250 mOsm/kg) to about 325 mOsM (or 325 mOsm/kg), about 250 mOsM (or 250 mOsm/kg) to about 300 mOsM (or 300 mOsm/kg), about 250 mOsM (or 250 mOsm/kg) to about 275 mOsM (or 275 mOsm/kg), about 275 mOsM (or 275 mOsm/kg) to about 325 mOsM (or 325 mOsm/kg), about 275 mOsM (or 275 mOsm/kg) to about 300 mOsM (or 300 mOsm/kg), or about 300 mOsM (or 300 mOsm/kg) to about 325 mOsM (or 325 mOsm/kg).

In various embodiments, the liquid pharmaceutical formulations described herein are isosmotic.

In various embodiments, the liquid pharmaceutical formulations described herein further comprise a second therapeutic agent. In certain embodiments, furosemide is the sole therapeutic agent present in the liquid pharmaceutical formulations described herein.

In various embodiments, a liquid pharmaceutical formulation useful in the treatment of a condition, disease, or disorder described herein is a liquid pharmaceutical formulation described in U.S. Patent No. 9,884,039.

### Methods of Treatment

In one aspect, the liquid pharmaceutical formulations described herein may be used for the treatment or prevention of a variety of conditions such as, but not limited to, congestion due to fluid overload, edema, and hypertension in a patient in need thereof. In various embodiments, the condition is selected from the group consisting of congestion due to fluid overload, edema, and hypertension, and combinations thereof. In certain embodiments, the condition is congestion due to fluid overload. In certain embodiments, the condition is edema. In certain embodiments, the condition is hypertension.

In certain embodiments, the patient is a human.

In various embodiments, provided herein is a method of treating a condition selected from the group consisting of congestion due to fluid overload, edema, and hypertension, in a human in need thereof, comprising administering subcutaneously to the human a total volume of 10 mL of a liquid pharmaceutical formulation comprising 80 mg furosemide over a period of about 0.5 hours to about 2 hours.

In certain embodiments, a method described herein, about 2 hours after administration is initiated, results in a total urine output of about 450 mL to about 950 mL, about 500 mL to about 950 mL, about 550 mL to about 950 mL. about 600 mL to about 950 mL, about 650 mL to about 950 mL, about 700 mL to about 950 mL, about 750 mL to about 950 mL, about 800 mL to about 950 mL, about 850 mL to about 950 mL, about 900 mL to about 950 mL, about 450 mL to about 900 mL, about 450 mL to about 850 mL, about 450 mL to about 800 mL, about 450 mL to about 750 mL, about 450 mL to about 700 mL, about 450 mL to about 650 mL, about 450 mL to about 600 mL, about 450 mL to about 550 mL, about 450 mL to about 500 mL, about 500 mL to about 900 mL, about 500 mL to about 850 mL, about 500 mL to about 800 mL, about 500 mL to about 750 mL, about 500 mL to about 700 mL, about 500 mL to about 650 mL, about 500 mL to about 600 mL, about 500 mL to about 550 mL, about 550 mL to about 900 mL, about 550 mL to about 850 mL, about 550 mL to about 800 mL, about 550 mL to about 750 mL, about 550 mL to about 700 mL, about 550 mL to about 650 mL, about 550 mL to about 600 mL, about 600 mL to about 900 mL, about 600 mL to about 850 mL, about 600 mL to about 800 mL, about 600 mL to about 750 mL, about 600 mL to about 700 mL, about 600 mL to about 650 mL, about 650 mL to about 900 mL, about 650 mL to about 850 mL, about 650 mL to about 800 mL, about 650 mL to about 750 mL, about 650 mL to about 700 mL, about 700 mL to about 900 mL, about 700 mL to about 850 mL, about 700 mL to about 800 mL, about 700 mL to about 750 mL, about 750 mL to about 900 mL, about 750 mL to about 850 mL. about 750 mL to about 800 mL, about 800 mL to about 900 mL, about 800 mL to about 850 mL. or about 850 mL to about 900 mL. In certain embodiments, a method described herein, about 2 hours after administration is initiated, results in a total urine output of about 450 mL to about 950 mL.

In certain embodiments, a method described herein, about 2 hours after administration is initiated, results in a total urine output of about 450 mL, about 500 mL, about 550 mL, about 600 mL, about 650 mL, about 700 mL, about 750 mL, about 800 mL, about 850 mL, about 900 mL. or about 950 mL.

In certain embodiments, a method described herein, about 6 hours after administration is initiated, results in a total urine output of about 2100 mL to about 2600 mL, about 2150 mL to about 2600 mL, about 2200 mL to about 2600 mL, about 2250 mL to about 2600 mL. about 2300 mL to about 2600 mL, about 2350 mL to about 2600 mL, about 2400 mL to about 2600 mL, about 2450 mL to about 2600 mL, about 2500 mL to about 2600 mL. about 2550 mL to about 2600 mL, about 2100 mL to about 2550 mL, about 2100 mL to about 2500 mL, about 2100 mL to about 2450 mL, about 2100 mL to about 2400 mL, about 2100 mL to about 2350 mL, about 2100 mL to about 2300 mL, about 2100 mL to about 2250 mL, about 2100 mL to about 2200 mL, about 2100 mL to about 2150 mL, about 2150 mL to about 2550 mL, about 2150 mL to about 2500 mL, about 2150 mL to about 2450 mL, about 2150 mL to about 2400 mL, about 2150 mL to about 2350 mL, about 2150 mL to about 2300 mL, about 2150 mL to about 2250 mL, about 2150 mL to about 2200 mL, about 2200 mL to about 2550 mL, about 2200 mL to about 2500 mL, about 2200 mL to about 2450 mL, about 2200 mL to about 2400 mL, about 2200 mL to about 2350 mL, about 2200 mL to about 2300 mL, about 2200 mL to about 2250 mL, about 2250 mL to about 2550 mL, about 2250 mL to about 2500 mL, about 2250 mL to about 2450 mL, about 2250 mL to about 2400 mL, about 2250 mL to about 2350 mL, about 2250 mL to about 2300 mL, about 2300 mL to about 2550 mL. about 2300 mL to about 2500 mL, about 2300 mL to about 2450 mL, about 2300 mL to about 2400 mL, about 2300 mL to about 2350 mL, about 2350 mL to about 2550 mL, about 2350 mL to about 2500 mL, about 2350 mL to about 2450 mL, about 2350 mL to about 2400 mL, about 2400 mL to about 2550 mL, about 2400 mL to about 2500 mL, about 2400 mL to about 2450 mL, about 2450 mL to about 2550 mL, about 2450 mL to about 2500 mL, or about 2500 mL to about 2550 mL. In certain embodiments, a method described herein, about 6 hours after administration is initiated, results in a total urine output of about 2100 mL to about 2600 mL.

In certain embodiments, a method described herein, about 6 hours after administration is initiated, results in a total urine output of about 2100 mL, about 2150 mL, about 2200 mL, about 2250 mL, about 2300 mL, about 2350 mL, about 2400 mL, about 2450 mL, about 2500 mL, about 2550 mL, or about 2600 mL.

In certain embodiments, a method described herein, about 24 hours after administration is initiated, results in a total urine output of about 3200 mL to about 4200 mL, about 3300 mL to about 4200 mL, about 3400 mL to about 4200 mL, about 3500 mL to about 4200 mL, about 3600 mL to about 4200 mL, about 3700 mL to about 4200 mL, about 3800 mL to about 4200 mL, about 3900 mL to about 4200 mL, about 4000 mL to about 4200 mL, about 4100 mL to about 4200 mL, about 3200 mL to about 4100 mL, about 3200 mL to about 4000 mL, about 3200 mL to about 3900 mL, about 3200 mL to about 3800 mL, about 3200 mL to about 3700 mL, about 3200 mL to about 3600 mL, about 3200 mL to about 3500 mL, about 3200 mL to about 3400 mL, about 3200 mL to about 3300 mL, about 3300 mL to about 4100 mL, about 3300 mL to about 4000 mL, about 3300 mL to about 3900 mL, about 3300 mL to about 3800 mL, about 3300 mL to about 3700 mL, about 3300 mL to about 3600 mL, about 3300 mL to about 3500 mL, about 3300 mL to about 3400 mL, about 3400 mL to about 4100 mL, about 3400 mL to about 4000 mL, about 3400 mL to about 3900 mL, about 3400 mL to about 3800 mL, about 3400 mL to about 3700 mL, about 3400 mL to about 3600 mL, about 3400 mL to about 3500 mL, about 3500 mL to about 4100 mL, about 3500 mL to about 4000 mL, about 3500 mL to about 3900 mL, about 3500 mL to about 3800 mL, about 3500 mL to about 3700 mL, about 3500 mL to about 3600 mL, about 3600 mL to about 4100 mL. about 3600 mL to about 4000 mL, about 3600 mL to about 3900 mL, about 3600 mL to about 3800 mL, about 3600 mL to about 3700 mL, about 3700 mL to about 4100 mL, about 3700 mL to about 4000 mL, about 3700 mL to about 3900 mL, about 3700 mL to about 3800 mL, about 3800 mL to about 4100 mL, about 3800 mL to about 4000 mL, about 3800 mL to about 3900 mL, about 3900 mL to about 4100 mL, about 3900 mL to about 4000 mL, or about 4000 mL to about 4100 mL. In certain embodiments, a method described herein, about 24 hours after administration is initiated, results in a total urine output of about 3200 mL to about 4200 mL.

In certain embodiments, a method described herein, about 24 hours after administration is initiated, results in a total urine output of about 3200 mL, about 3300 mL, about 3400 mL, about 3500 mL, about 3600 mL, about 3700 mL, about 3800 mL, about 3900 mL, about 4000 mL, about 4100 mL, or about 4200 mL.

In certain embodiments, a method described herein, about 2 hours after administration is initiated, results in a total urine sodium excretion of about 40 mmol to about 110 mmol, about 50 mmol to about 110 mmol, about 60 mmol to about 110 mmol, about 70 mmol to about 110 mmol, about 80 mmol to about 110 mmol, about 90 mmol to about 110 mmol, about 100 mmol to about 110 mmol, about 40 mmol to about 100 mmol, about 40 mmol to about 90 mmol, about 40 mmol to about 80 mmol, about 40 mmol to about 70 mmol, about 40 mmol to about 60 mmol, about 40 mmol to about 50 mmol, about 50 mmol to about 100 mmol, about 50 mmol to about 90 mmol, about 50 mmol to about 80 mmol, about 50 mmol to about 70 mmol, about 50 mmol to about 60 mmol, about 60 mmol to about 90 mmol, about 60 mmol to about 80 mmol, about 60 mmol to about 70 mmol, about 70 mmol to about 100 mmol, about 70 mmol to about 90 mmol, about 70 mmol to about 80 mmol, about 80 mmol to about 100 mmol, about 80 mmol to about 90 mmol, or about 90 mmol to about 100 mmol. In certain embodiments, a method described herein, about 2 hours after administration is initiated, results in a total urine sodium excretion of about 40 mmol to about 110 mmol.

In certain embodiments, a method described herein, about 2 hours after administration is initiated, results in a total urine sodium excretion of about 40 mmol, about 50 mmol, about 60 mmol, about 70 mmol, about 80 mmol, about 90 mmol, about 100 mmol, or about 110 mmol.

In certain embodiments, a method described herein, about 6 hours after administration is initiated, results in a total urine sodium excretion of about 220 mmol to about 300 mmol, about 230 mmol to about 300 mmol, about 240 mmol to about 300 mmol, about 250 mmol to about 300 mmol, about 260 mmol to about 300 mmol, about 270 mmol to about 300 mmol, about 280 mmol to about 300 mmol, about 290 mmol to about 300 mmol, about 220 mmol to about 290 mmol, about 220 mmol to about 280 mmol, about 220 mmol to about 270 mmol, about 220 mmol to about 260 mmol, about 220 mmol to about 250 mmol, about 220 mmol to about 240 mmol, about 220 mmol to about 230 mmol, about 230 mmol to about 290 mmol, about 230 mmol to about 280 mmol, about 230 mmol to about 270 mmol, about 230 mmol to about 260 mmol, about 230 mmol to about 250 mmol, about 230 mmol to about 240 mmol, about 240 mmol to about 290 mmol, about 240 mmol to about 280 mmol, about 240 mmol to about 270 mmol, about 240 mmol to about 260 mmol, about 240 mmol to about 250 mmol, about 250 mmol to about 290 mmol, about 250 mmol to about 280 mmol, about 250 mmol to about 270 mmol, about 250 mmol to about 260 mmol, about 260 mmol to about 290 mmol, about 260 mmol to about 280 mmol, about 260 mmol to about 270 mmol, about 270 mmol to about 290 mmol, about 270 mmol to about 280 mmol, or about 280 mmol to about 290 mmol. In certain embodiments, a method described herein, about 6 hours after administration is initiated, results in a total urine sodium excretion of about 220 mmol to about 300 mmol.

In certain embodiments, a method described herein, about 6 hours after administration is initiated, results in a total urine sodium excretion of about 220 mmol, about 230 mmol, about 240 mmol, about 250 mmol, about 260 mmol, about 270 mmol, about 280 mmol, about 290 mmol, or about 300 mmol.

In certain embodiments, a method described herein, about 24 hours after administration is initiated, results in a total urine sodium excretion of about 350 mmol to about 480 mmol, about 360 mmol to about 480 mmol, about 370 nunol to about 480 mmol, about 380 mmol to about 480 mmol, about 390 mmol to about 480 mmol, about 400 mmol to about 480 mmol, about 410 mmol to about 480 mmol, about 420 mmol to about 480 mmol, about 430 mmol to about 480 mmol, about 440 mmol to about 480 mmol, about 450 mmol to about 480 mmol, about 460 mmol to about 480 mmol, about 470 mmol to about 480 mmol, about 350 mmol to about 470 mmol, about 350 mmol to about 460 mmol, about 350 mmol to about 450 mmol, about 350 mmol to about 440 mmol, about 350 mmol to about 430 mmol, about 350 mmol to about 420 mmol, about 350 mmol to about 410 mmol, about 350 mmol to about 400 mmol, about 350 mmol to about 390 mmol, about 350 mmol to about 380 mmol, about 350 mmol to about 370 mmol, about 350 mmol to about 360 mmol, about 360 mmol to about 470 mmol, about 360 mmol to about 460 mmol, about 360 mmol to about 450 mmol, about 360 mmol to about 440 mmol, about 360 mmol to about 430 mmol, about 360 mmol to about 420 mmol, about 360 mmol to about 410 mmol, about 360 mmol to about 400 mmol, about 360 mmol to about 390 mmol, about 360 mmol to about 380 mmol, about 360 mmol to about 370 mmol, about 370 mmol to about 470 mmol, about 370 nunol to about 460 mmol, about 370 mmol to about 450 mmol, about 370 mmol to about 440 mmol, about 370 mmol to about 430 mmol, about 370 mmol to about 420 mmol, about 370 mmol to about 410 mmol, about 370 mmol to about 400 mmol, about 370 mmol to about 390 mmol, about 370 mmol to about 380 mmol, about 380 mmol to about 470 mmol, about 380 mmol to about 460 mmol, about 380 mmol to about 450 mmol, about 380 mmol to about 440 mmol, about 380 mmol to about 430 mmol, about 380 mmol to about 420 mmol, about 380 mmol to about 410 mmol, about 380 mmol to about 400 mmol, about 380 mmol to about 390 mmol, about 390 mmol to about 470 mmol, about 390 mmol to about 460 mmol, about 390 mmol to about 450 mmol, about 390 mmol to about 440 mmol, about 390 mmol to about 430 mmol, about 390 mmol to about 420 mmol, about 390 mmol to about 410 mmol, about 390 mmol to about 400 mmol, about 400 mmol to about 470 mmol, about 400 mmol to about 460 mmol, about 400 mmol to about 450 mmol, about 400 mmol to about 440 mmol, about 400 mmol to about 430 mmol, about 400 mmol to about 420 mmol, about 400 mmol to about 410 mmol, about 410 mmol to about 470 mmol, about 410 mmol to about 460 mmol, about 410 mmol to about 450 mmol, about 410 mmol to about 440 mmol, about 410 mmol to about 430 mmol, about 410 mmol to about 420 mmol, about 420 mmol to about 470 mmol, about 420 mmol to about 460 mmol, about 420 mmol to about 450 mmol, about 420 mmol to about 440 mmol, about 420 mmol to about 430 mmol, about 430 mmol to about 470 mmol, about 430 mmol to about 460 mmol, about 430 mmol to about 450 mmol, about 430 mmol to about 440 mmol, about 440 mmol to about 470 mmol, about 440 mmol to about 460 mmol, about 440 mmol to about 450 mmol, about 450 mmol to about 470 mmol, about 450 mmol to about 460 mmol, or about 460 mmol to about 470 mmol. In certain embodiments, a method described herein, about 24 hours after administration is initiated, results in a total urine sodium excretion of about 350 mmol to about 480 mmol.

In certain embodiments, a method described herein, about 24 hours after administration is initiated, results in a total urine sodium excretion of about 350 mmol, about 360 mmol, about 370 mmol, about 380 mmol, about 390 mmol, about 400 mmol, about 410 mmol, about 420 mmol, about 430 mmol, about 440 mmol, about 450 mmol, about 460 mmol, about 470 mmol, or about 480 mmol.

In various embodiments, provided herein is a method of treating a condition selected from the group consisting of congestion due to fluid overload, edema, and hypertension, in a human in need thereof, comprising administering subcutaneously to the human a liquid pharmaceutical formulation comprising furosemide over a period of about 0.5 hours to about 2 hours, wherein one or more of the following:
the method, about 2 hours after administration is initiated, results in a total urine output of about 450 mL to about 950 mL;
the method, about 6 hours after administration is initiated, results in a total urine output of about 2100 mL to about 2600 mL;
the method, about 24 hours after administration is initiated, results in a total urine output of about 3200 mL to about 4200 mL;
the method, about 2 hours after administration is initiated, results in a total urine sodium excretion of is about 40 mmol to about 110 mmol;
the method, about 6 hours after administration is initiated, results in a total urine sodium excretion of about 220 mmol to about 300 mmol; and
the method, about 24 hours after administration is initiated, results in a total urine sodium excretion of about 350 mmol to about 480 mmol.

In certain embodiments, the human is administered a total volume of about 8 mL to about 12 mL, about 8.5 mL to about 12 mL, about 9 mL to about 12 mL, about 9.5 mL to about 12 mL, about 10 mL to about 12 mL, about 10.5 mL to about 12 mL, about 11 mL to about 12 mL, about 11.5 mL to about 12 mL, about 8 mL to about 11.5 mL, about 8 mL to about 11 mL, about 8 mL to about 10.5 mL, about 8 mL to about 10 mL, about 8 mL to about 9.5 mL, about 8 mL to about 9 mL, about 8 mL to about 8.5 mL, about 8.5 mL to about 11.5 mL, about 8.5 mL to about 11 mL, about 8.5 mL to about 10.5 mL, about 8.5 mL to about 10 mL, about 8.5 mL to about 9.5 mL, about 8.5 mL to about 9 mL, about 9 mL to about 11.5 mL, about 9 mL to about 11 mL, about 9 mL to about 10.5 mL, about 9 mL to about 10 mL, about 9 mL to about 9.5 mL, about 9.5 mL to about 11.5 mL, about 9.5 mL to about 11 mL, about 9.5 mL to about 10.5 mL, about 9.5 mL to about 10 mL, about 10 mL to about 11.5 mL, about 10 m L to about 11 mL. about 10 mL to about 10.5 mL, about 10.5 mL to about 11.5 mL, about 10.5 mL to about 11 mL, or about 11 mL to about 11.5 mL of the liquid pharmaceutical formulation.

In certain embodiments, the human is administered a total volume of about 8 mL, about 8.5 mL. about 9 mL. about 9.5 mL, about 10 mL, about 10.5 mL, about 11 mL, about 11.5 mL. or about 12 mL of the liquid pharmaceutical formulation. In certain embodiments, the human is administered a total volume of about 10 mL of the liquid pharmaceutical formulation.

In various embodiments, provided herein is a method of treating a condition selected from the group consisting of congestion due to fluid overload, edema, and hypertension, in a human in need thereof, comprising administering subcutaneously to the human a total volume of 10 mL of a liquid pharmaceutical formulation comprising 80 mg furosemide over a period about 0.5 hours to about 2 hours, wherein one or more of the following:
the method, about 2 hours after administration is initiated, results in a total urine output of about 2100 mL to about 2600 mL;
the method, about 6 hours after administration is initiated, results in a total urine output of about 2100 mL to about 2600 mL;
the method, about 24 hours after administration is initiated, results in a total urine output of about 3200 mL to about 4200 mL;
the method, about 2 hours after administration is initiated, results in a total urine sodium excretion of about 40 mmol to about 110 mmol;
the method, about 6 hours after administration is initiated, results in a total urine sodium excretion of about 220 mmol to about 300 mmol: and
the method, about 24 hours after administration is initiated, results in a total urine sodium excretion of about 350 mmol to about 480 mmol.

In certain embodiments, administering the liquid pharmaceutical formulation is over a period of about 0.5 hours to about 1 hour, about 0.5 hours to about 1.5 hours, about 0.5 hours to about 2 hours. In certain embodiments, administering the liquid pharmaceutical formulation is over a period of about 0.5 hours to about 2 hours.

In certain embodiments, administering the liquid pharmaceutical formulation comprises a continuous delivery profile over a period of about 0.5 hours, about 1 hour, about 1.5 hours, about 2 hours. In certain embodiments, administering the liquid pharmaceutical formulation comprises a continuous delivery profile over a period of about two hours. In certain embodiments, administering the liquid pharmaceutical formulation comprises a continuous delivery profile over a period of about 1.5 hours In certain embodiments, administering the liquid pharmaceutical formulation comprises a continuous delivery profile over a period of about one hour. In certain embodiments, administering the liquid pharmaceutical formulation comprises a continuous delivery profile over a period of about 0.5 hours.

In certain embodiments, administering the liquid pharmaceutical formulation comprises a bi-phasic delivery profile over a period of about one hour, about two hours, about three hours, or about four hours. In certain embodiments, administering the liquid pharmaceutical formulation comprises a bi-phasic delivery profile over a period of about two hours In some embodiments, the bi-phasic delivery profile results in the delivery of about 40 mg furosemide over about the first 0.5 hour of the period of two hours followed by a 1-hour pause in delivery, and about 40 mg furosemide over about the last 0.5 hour of the period of two hours.

In certain embodiments, the liquid pharmaceutical formulation is administered from a prefilled polymeric cartridge. In certain embodiments, the polymeric prefilled cartridge is associated with an on-body wearable delivery device. In certain embodiments, the polymeric cartridge of the polymeric prefilled cartridge comprises a cyclic olefin polymer. In certain embodiments, the polymeric prefilled cartridge is a single-use cartridge.

In certain embodiments, a method described herein, during administrating of the liquid pharmaceutical formulation, results in a pain score of 0, wherein the pain score is measured using an 11-point numeric pain assessment scale. In certain embodiments, a method described herein, nearly immediately after administration of the liquid pharmaceutical formulation is complete, results in a pain score of 0, wherein the pain score is measured using an 11-point numeric pain assessment scale. For example, an 11-point numeric pain assessment scale is scored over a range of 0 to 10 in whole integer increments, with 0 being equivalent to no pain and 10 being equivalent to the worst possible pain. See, e.g., Kwong and Pathak, "Validation of the eleven-point pain scale in the measurement of migraine headache pain," Cephalalgia, 27(4), 336-342 (2007).

In certain embodiments, the human is an adult human. In certain embodiments, the adult human has New York Heart Association (NYHA) Class II. Class III or Class IV heart failure, liver failure, or renal failure. In certain embodiments, the adult human displays reduced responsiveness to oral diuretics before beginning subcutaneous administration according to the method.

In certain embodiments, the adult human is 30 to 80 years of age, 35 to 80 years of age, 40 to 80 years of age, 45 to 80 years of age, 50 to 80 years of age. 55 to 80 years of age, 60 to 80 years of age, 65 to 80 years of age, 70 to 80 years of age, 75 to 80 years of age, 30 to 75 years of age, 30 to 70 years of age, 30 to 65 years of age, 30 to 60 years of age, 30 to 55 years of age. 30 to 50 years of age, 30 to 45 years of age. 30 to 40 years of age, 30 to 35 years of age. 35 to 75 years of age, 35 to 70 years of age, 35 to 65 years of age, 35 to 60 years of age, 35 to 55 years of age, 35 to 50 years of age, 35 to 45 years of age, 35 to 40 years of age, 40 to 75 years of age, 40 to 70 years of age. 40 to 65 years of age, 40 to 60 years of age. 40 to 55 years of age, 40 to 50 years of age, 40 to 45 years of age, 45 to 75 years of age, 45 to 70 years of age, 45 to 65 years of age, 45 to 60 years of age, 45 to 55 years of age, 45 to 50 years of age, 50 to 75 years of age, 50 to 70 years of age, 50 to 65 years of age. 50 to 60 years of age, 50 to 55 years of age. 55 to 75 years of age, 55 to 70 years of age. 55 to 65 years of age, 55 to 60 years of age, 60 to 75 years of age, 60 to 70 years of age, 60 to 65 years of age, 65 to 75 years of age, 65 to 70 years of age, or 70 to 75 years of age. In certain embodiments, the adult human is 45 to 80 years of age.

In certain embodiments, the adult human is 30, 31, 32, 33, 34, 35, 36, 37, 38, 39, 38. 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 years of age

In certain embodiments, the edema is associated with congestive heart failure, cirrhosis of the liver or renal disease. In certain embodiments, the renal disease is nephrotic syndrome.

### Kits

In one aspect, the invention provides kits for the treatment or prevention of a variety of conditions such as, but not limited to, congestion due to fluid overload, edema, and hypertension in a patient in need thereof. In certain embodiments, the condition, disease, or disorder is selected from the group consisting of congestion due to fluid overload, edema, and hypertension. In certain embodiments, the condition is congestion due to fluid overload. In certain embodiments, the condition is edema. In certain embodiments, the condition is hypertension.

In various embodiments, the invention provides a polymeric prefilled cartridge comprising a liquid pharmaceutical formulation described herein.

In various embodiments, the polymeric prefilled cartridge is associated with an on-body wearable delivery device.

In various embodiments, the polymeric prefilled cartridge is a single-use cartridge.

In various embodiments, a single-use polymeric prefilled cartridge that can be associated with an on-body wearable delivery device for administering subcutaneously a liquid pharmaceutical formulation to an adult human, wherein the single-use polymeric prefilled cartridge comprises the liquid pharmaceutical formulation and the liquid pharmaceutical formulation is isosmotic, has a pH between about 7 to about 7.8. and consists of:
80 mg furosemide:
about 79 mg tromethamine hydrochloride:
optionally, one or more other pharmaceutically acceptable excipients; and
water to a total volume of 10 mL.

In various embodiments, the one or more other pharmaceutically acceptable excipients comprises sodium chloride, sodium hydroxide, hydrochloric acid. or combinations thereof.

In various embodiments, a kit includes instructions for use, for example, of a prefilled polymeric cartridge and an on-body wearable delivery device.

### EXAMPLES

The invention now being generally described, will be more readily understood by reference to the following examples, which are merely for the purposes of illustration of certain aspects and embodiments of the present invention, and are not intended to limit the invention.

### Example 1. Manufacturing Process for a Furosemide Formulation (8 mg/mL)

In the following example, the manufacturing process for preparing a 8 mg/mL furosemide liquid formulation is described.

The excipients (sodium chloride and tromethamine hydrochloride) and drug substance Furosemide USP/EP were dispensed into individual containers in amounts necessary to achieve final concentrations of furosemide, sodium chloride, and tromethamine hydrochloride of 8 mg/mL, 5.84 mg/mL, and 7.88 mg/mL, respectively.

The specified amount of water for injection (WFI) and the excipients, tromethamine hydrochloride and sodium chloride, were added to an appropriately sized mixing vessel. The vessel was closed, and the excipients were mixed. Sodium hydroxide (NaOH) solution was added to the vessel and mixed.

A pH sample was taken, and the solution adjusted with hydrochloric acid (HCl) or additional NaOH to a pH of 8.50 ± 0.10, if necessary. If an adjustment was necessary, the solution was mixed after each addition before sampling again.

The furosemide USP/NF/EP was then added to the excipient solution followed by a WFI rinse of the dispensing container. The vessel was closed, a lid was placed on the vessel to protect the drug substance from light, and the solution was mixed. After mixing, if the API had not dissolved, NaOH solution was added to the solution to facilitate dissolution.

A pH sample was taken, and the solution adjusted with HCl or additional NaOH solution to a pH of 7.40 ± 0.10.

After pH adjustments. WFI was added to the resulting solution to achieve the final weight. Samples were collected after final mixing for assay, pH, osmolality and appearance testing before continuing to filtration.

The furosemide liquid formulation was first filtered through a 0.45 µm bioburden reduction filter, and then a sterile filtration through dual 0.22 µm was performed. The furosemide liquid formulation was then filled into 10 mL polymeric cartridges and stoppered using an aseptic process.

### Example 2. Furosemide Pharmacokinetic-Pharmacodynamic Modeling Study

### Study Objective

To perform pharmacokinetic (PK) and pharmacodynamic (PD) modeling and simulation utilizing PK and PD data from Study scP-01-002. Study scP-01-002 provided PK and PD data that demonstrated a bridge between furosemide administered as subcutaneous (SC) infusion to the listed drug (LD) administered as intravenous (IV) injection.

The purpose of this PK-PD modeling and simulation was to construct a population PK model to describe the time course of furosemide systemic exposure in patients with congestive heart failure and to investigate sources of variability in patient exposure to furosemide. In addition, an assessment of the likelihood of SC infusion administration being bioequivalence (BE) to the LD, IV furosemide with various dosing regimens was performed.

### Tasks for Study

### Task 1: Pharmacokinetic Analysis

Conduct a noncompartmental (NCA) PK analysis for partial AUCs (area under the plasma concentration vs. time profile) and partial AUECs (area under the effective (PD) vs. time profile) of the PK Study scP-01-002.

### Task 2: Population Pharmacokinetic Modeling y

Conduct population PK modeling to identify the SC absorption rate from the best fit model for SC furosemide. A covariate model will assess the effect of covariates (e.g., age, bodyweight and renal function) on the population PK parameters (e.g., volume of distribution, clearance, and rate of subcutaneous absorption).

### Task 3: Pharmacokinetic Simulations for Furosemide Subcutaneous Delivery

Conduct PK simulation for subcutaneous delivery of 80 mg (total) of furosemide over different dosing intervals and infusion durations. A total of 5 BE trials were simulated to investigate the likelihood of demonstrating BE between SC furosemide infusion (Test) vs IV furosemide injection (LD, Reference) with various dosing regimens. All BE trials used observed data from the IV furosemide injection (LD, Reference) obtained from Study scP-01-002.

Data from the SC treatment arm (Test arm) were simulated for the followings:
- **BE Trial 1:** Test: 40 mg SC infusion × 2 doses (over 30 minutes): at time 0 and 1.5 hour
- **BE Trial 2:** Test: 80 mg SC infusion over 1 hour
- **BE Trial 3:** Test: 80 mg SC infusion over 2 hours
- **BE Trial 4:** Test: 80 mg SC infusion over 4 hours
- **BE Trial 5:** Test: 80 mg SC infusion over 8 hours

### Task 4: Pharmacokinetic Simulation for Virtual Bioequivalence Trial

Conduct PK simulation for virtual BE trials comparing SC delivery of furosemide with a different drug delivery schedule (i.e., dose, time of infusion). Compare 5 SC infusion administrations to the PK of an IV delivery (fixed drug delivery dose/infusion time as listed in the labeling of the LD, NDA 018667; and observed data from scP-01-002).

### Task 5: Population PK-PD Modeling

Conduct population PK-PD modeling to establish the PK-PD model for the SC furosemide formulation and identify the PK parameters that drive PD effect. The final PK-PD model was used to simulate the cumulative urine volume output for each trial comparing SC and IV treatments.

### High-Level Summary

Task 1: BE assessment on AUC₀₋₂, AUC₀₋₄, AUC₀₋₆, AUC₀₋₈, AUCₗₐₛₜ and AUC_{inf} between SC infusion and IV treatment arms demonstrated that SC infusion, administered as outlined in the Study scP-01-002, is BE to the IV, LD on AUC₀₋₈; AUCₗₐₛₜ and AUC_{inf}. However, AUC₀₋₂, AUC₀₋₄ and AUC₀₋₆ for the SC infusion treatment arm fell outside on the lower bound of the 80.00%-125.00% BE limits compared to the IV treatment arm. Further explanation is presented under the summary for Task 3 and Task 4 below.

Task 2: A population PK model to describe the time course of furosemide systemic exposure in patients with chronic heart failure was constructed. Further, sources of variability in patient exposure to furosemide was investigated. A two compartment PK model with the first order subcutaneous absorption rate (ka) and first order elimination rate best described the PK profile of furosemide.

The covariates, creatine clearance and body weight, were found to impact patient furosemide exposure Creatine clearance was found to have a significant effect on furosemide clearance while body weight had a significant effect on furosemide volume of distribution. Age did not show significant effect on any of the population PK parameters.

Task 3 and 4: An assessment of the likelihood of SC infusion administration with various dosing regimens being BE to the LD, IV furosemide was performed. The PK BE criteria were AUCₗₐₛₜ and AUC_{0-inf}. AUC₀₋₂, AUC₀₋₄, AUC₀₋₆ and AUC₀₋₈ were not used to assess overall BE, however, they were used to develop the PK-PD model for urine output. The final constructed population PK model was used to simulate individual furosemide plasma concentration vs time profiles of the SC infusion administration with different dosing regimens.

Bioequivalence for AUCₗₐₛₜ and AUC_{inf} was demonstrated between the SC furosemide infusion and LD IV furosemide for all tested dosing regimens.

The likelihood of the BE criteria being achieved generally decreased with increasing infusion times. The only regime to achieve the BE criteria relative to the IV LD for AUC₀₋₂ was 80 mg SC over 1 hour. AUC₀₋₅ and AUC₀₋₁ were not valuated in this analysis. This observation is consistent with the clinical utility of subcutaneous furosemide being limited to non-emergent situations where the IV LD should be utilized.

Bioequivalence was demonstrated between the SC infusion and LD, IV furosemide for pAUCs (0-6, 0-8, last and inf) tested when 80 mg furosemide was delivered as 40 mg SC × 2 doses (over 30 minutes. Trial 1) and 80 mg delivered over 1 or 2 hours (Trials 2 and 3). AUC₀₋₆ was outside the 80.00-125.00% BE limits on the lower bound when 80 mg furosemide was delivered over a longer infusion time (over 4 hours or 8 hours, Trials 4 and 5) versus shorter infusion time (over 1, and 2 hours, Trials 1, 2 and 3).

As both furosemide half-life of subcutaneous absorption and terminal elimination are short, to obtain the comparable early partial AUC between IV and SC infusion (e.g., 6- or 8-hour post dose) the 80 mg total dose SC infusion should be administered over a shorter infusion time. Among tested SC infusion dosing regimens, a total of 80 mg administered over a shorter period (1hour or 2 hour) demonstrated comparable AUC₀₋₆ between the SC infusion and IV. However, when a total of 80 mg SC infusion was administered over a longer period (4 hour (trial 4), 5 hours (as administered in scP-01-002), and 8 hours (trial)). the AUC₀₋₆ was outside 80.00-125.00% BE limits on the lower bound, compared to the LD IV treatment.

Task 5: The population PK-PD model described provides the best fit to the PK-PD data. The PK and PD data were modelled sequentially. The final population PK model with covariate effects identified in Task 2 was used to simulate furosemide excretion rate into urine. The simulated furosemide excretion rate into urine was then used in the indirect-response PD model to establish the PK-PD relationship for furosemide. The PK-PD model was used to estimate IC₅₀, the furosemide excretion rate into urine that provide 50% of the maximum PD effect (urine volume excretion rate mL/hr). Note that as furosemide produces its pharmacologic effect via inhibition mechanism, IC₅₀ parameter terminology (not EC₅₀ which is used to describe the pharmacological effect produced by stimulation mechanism) was used herein.

An IC₅₀, furosemide excretion rate into urine (ng/hour) that provide 50% of maximum urine output (mL/hr); was found to be the main driver of the PD effect. Results of the PK-PD modeling and simulation provide insight into the understanding the relationship between the furosemide excretion rate to the urine and the rate of urine volume output in patients with chronic heart failure. Interpreting the furosemide plasma threshold level from the value of the IC₅₀ in the absence of clinically meaningful urine output volume is considered speculative. As a result, the cumulative urine output was simulated and compared between SC furosemide infusion and IV furosemide injection treatment in the 5 simulated trials:
- BE Trial 1: Test: 40 mg SC infusion × 2 doses (over 30 minutes); at time 0 and 1.5 hour.

The total urine volume output is similar to the IV treatment arm at 4 hours. By 6 hours to 24-hour post dose, SC treatment arm produced ~250 mL urine volume more than the IV treatment arm.
- BE Trial 2: Test: 80 mg SC infusion over 1 hour.

The total urine volume output is similar between the SC and IV treatment arms at all time points up to 24 hours.
- BE Trial 3: Test: 80 mg SC infusion over 2 hours.

The total urine volume output from the SC treatment arm is similar to the IV treatment by 4 hours post dose and slightly greater than the IV treatment by 150 mL at 24 hours post dose.
- BE Trial 4: Test: 80 mg SC infusion over 4 hours.

The total urine volume output from the SC treatment arm is similar to the IV treatment by 4 hours post dose. The SC treatment arm is estimated to produce 160 mL more than the urine volume output at 24 hours post dose compared to the IV treatment.
- BE Trial 5: Test: 80 mg SC infusion over 8 hours.

The total urine volume output from the IV treatment arm is ~800 mL greater than the IV treatment by 4 hours post dose. However, the SC treatment arm is estimated to produce approximately 1000 mL more than the urine volume output at 24 hours post dose compared to the IV treatment.

### Source of Data

Data source for the PK/PD modeling and simulation was obtained from Study scP-01-002 (ClinicalTrials.gov Identifier: NCT02329834). See also Sica et al., "Subcutaneous Furosemide in Heart Failure: Pharmacokinetic Characteristics of a Newly Buffered Solution," JACC Basic Transl. Sci. 3(1), 25-34 (2018). This study was an open-label, single-center, single-dose, randomized, two-way (two-period) crossover study designed to assess the PK and bioavailability of furosemide after SC and IV administration. Adult patients previously diagnosed with mild to moderate heart failure (NYHA class II/III) who were being treated concomitantly with oral furosemide therapy at a dose of ≥ 40 mg/day were enrolled. A total of 16 patients were randomly assigned to 1 of 2 treatment sequences. Patients discontinued oral furosemide at least 24 hours prior to administration of study drug for each crossover period.

### Study Descriptive

### Treatments arms:

- Intravenous Furosemide (IV Furosemide): Hospira, Furosemide Injection, Solution 10 mg/mL (NDA 018667), (total dose = 80 mg) administered intravenously 40 mg over 2 minutes by IV bolus injection followed by a second dose of 40 mg over 2 minutes 120 minutes later (reference treatment).
- Subcutaneous Furosemide (SC Furosemide): Furosemide Injection Solution, 8 mg/mL, (total dose = 80 mg dose) administered subcutaneously as 30 mg over the first hour and then as 12.5 mg per hour over the subsequent 4 hours (test treatment).

Plasma samples for furosemide concentration measurement:
- SC infusion treatment arm: 0 (pre-dose), 0.5, 1, 1.5, 2, 3, 4, 5, 5.08, 5.25, 5.5, 5.75, 6. 8, 10, 12. 14, 16. and 24 hours post-dose.
- IV injection treatment arm: 0, 0.083, 0.25, 0.5, 0.75, 1, 1.5, 2, 2.08, 2.25, 2.5, 2.75. 3, 3.5, 4, 5. 6, 8, 10. 12, 14, and 16 hours post-dose.

Urine samples for urinary sodium and total urine output volume measurement:
- Patients were instructed to void immediately before dosing (pre-dose urine) and at the end of each collection period. Urine was collected from spontaneous voids during time periods of (0-1), (1-2). (2-4), (4-6), (6-8), (8-10), (10-12), (12-18), (18-24) hours after initiation of dosing (time 0) for each treatment period. Note that the pre-dose urine sodium concentrations were not provided because the lab pre-dose samples were not acquired.

Of the 16 patients who completed both treatment periods, several subjects had quantifiable pre-dose concentrations of furosemide which were generally less than 5% of Cmax while one patient (Patient 2), exhibited a pre-dose concentration of 927.55 ng/mL (>35% of Cmax) prior to administration of the subcutaneous infusion furosemide treatment. Per FDA guidance FDA, 2014, Patient 2 was excluded from the primary noncompartmental parameter estimates (scP-01-02 study-report) and statistical analyses because the pre-dose concentration was greater than 5% of the subject's Cₘₐₓ. Therefore, the PK/PD modeling and simulation outlined herein was performed on 15 patients.

Urine volume and urine sodium were selected as the primary pharmacodynamic parameters for study scP-01-002.

### Methods

### PHARMACOKINETIC ANALYSIS -TASK 1

The following PK parameters were estimated using non-compartmental analysis (NCA) of plasma concentration time course data from study scP-01-002 to calculate AUCₗₐₛₜ and AUC_{inf}. In addition, the early partial AUCs (pAUCs), included AUC₀₋₂, AUC₀₋₄, AUC₀₋₆. AUC₀₋₈, AUC₀₋₁₂, and AUC₀₋₂₄ for both IV and SC treatment were also calculated for exploratory PK-PD relationship assessment.

Bioequivalence assessments on AUCₗₐₛₜ and AUC_{inf} were performed to compare the total furosemide exposure between SC and IV treatment arms from Study scP-01-002 data. Note that the BE assessments were also performed for Task 4. Comparisons were performed using a linear mixed model with fixed effects for sequence, period, and treatment, and a random effect for subjects nested in sequence. Least-squares geometric means for AUCₗₐₛₜ and AUC_{inf} along with 90% confidence interval (CI) were calculated for the ratio (SC vs IV) of the geometric means of the furosemide AUCₗₐₛₜ and AUC_{inf}. If the calculated 90% CIs for the AUCₗₐₛₜ and AUC_{inf} fell within the range of 80.00 - 125.00%, then BE is demonstrated for furosemide total systemic exposure between SC and IV treatments.

Phoenix WinNonlin version 6.4 (Pharsight, Mountain View, CA) was used for BE determination (90% CI) and the estimation of the PK parameters utilizing non-compartmental analysis (NCA).

The NCA PK analysis for partial AUECs (area under the effective (PD) vs. time profile) was planned in Task 1. The rate of excretion of urine volume and urine sodium were deemed more appropriate for the PK-PD relationship of furosemide (Jusko and Ko, 1994). Therefore, the AUEC, the urine volume rate of excretion vs time profile was expressed as mL/hr at each mid-point of the time duration specified above (i.e., 0.5, 1, 3, 5, 7, 9, 11, 15 and 21 hour) was used as data input in the sequential PK-PD modeling (Task 5).

### POPULATION PHARMACOKINETIC-PHARMACODYNAMIC MODELING AND SIMULATION TASKS 2-5

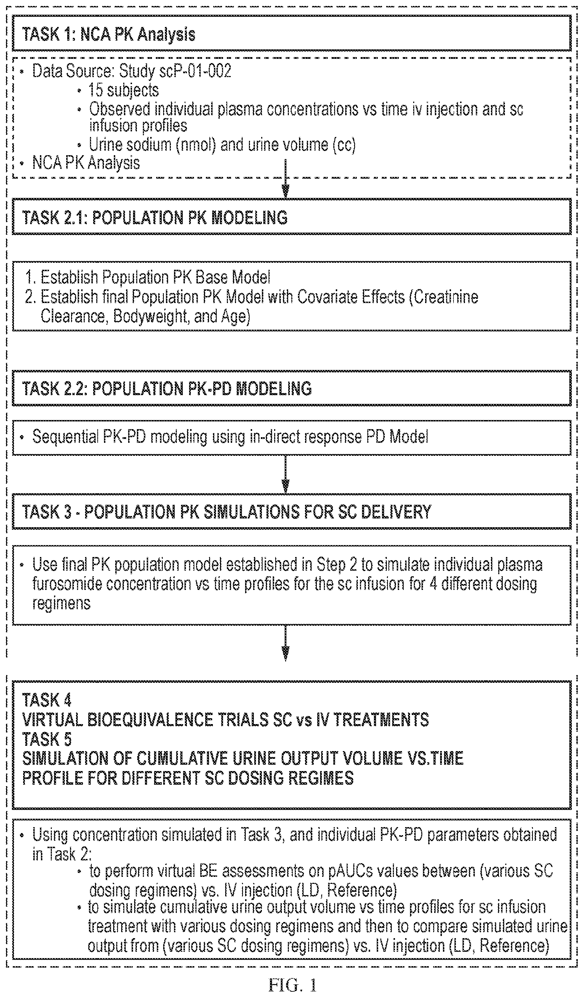

The PK-PD modeling and simulation tasks 1-5 performed herein are presented in FIG. 1.

### FINAL POPULATION PHARMACOKINETIC-PHARMACODYNAMIC MODEL TASK 5

### FINAL POPULATION PK-PD MODEL WITH COVARIATE EFFECTS

Population PK-PD modeling was performed to establish the PK-PD model for furosemide and to identify the PK parameters that drive PD effect (i.e., IC₅₀). An evaluation of the IC₅₀ of furosemide excretion rate into urine was performed via sequential population PK-PD modeling described herein.

Goodness-of fit for a linear regression analysis on rate of urine output vs urine sodium output was determined. A coefficient of determination for linear regression analysis R-squared value of 0.9587 was identified demonstrating the linear relationship between the rate of urine sodium output and rate of urine volume output. Both sodium output and urine volume output were considered primary PD endpoints of furosemide pharmacologic effect in study scP-01-002. This is in line with reports in the literature in which PD variables of furosemide-induced effect (i.e., urine flow rate, sodium excretion rate, and chloride excretion rate), have been shown to yield almost identical response patterns (Hammarlund et al., 1985: Hammarlund-Udenaes and Benet, 1989). Due to these identical response patterns, the rate of urine volume output was selected as the PD input for the PD model rather than urine sodium.

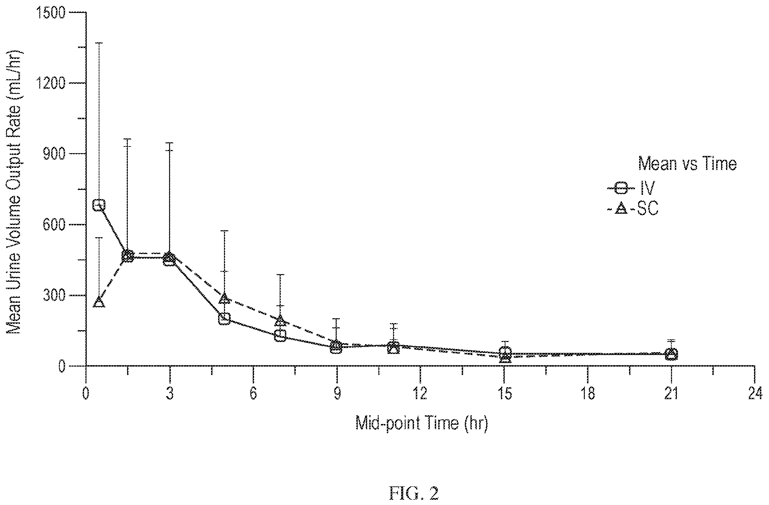

The AUEC, the urine volume rate of excretion vs time profile was expressed as mL/hr at each mid-point of the time duration specified above (i.e., 0.5, 1, 3, 5, 7. 9, 11, 15 and 21 hr), as shown in FIG. 2.

The goodness of fit of the population PK-PD model was evidenced by residual diagnostic plots:
- individual prediction (IPRED) vs. individual weighted residual (IWRES):
- dependent variable (observed urine volume. DV) vs. IPRED: and
- individual weighted residual vs. standard normal quantiles.

These diagnostic plots showed that, in general, the error model selected was reasonable to describe the data and adequately captured the observed PD data (urine volume output).

### MODEL VALIDATION OF THE FINAL POPULATION PK-PD MODEL

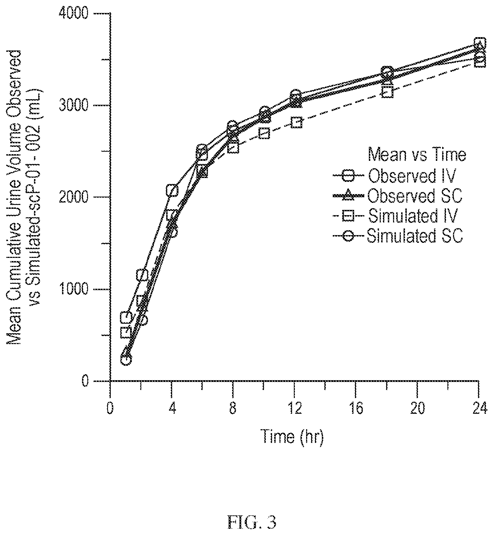

To validate the final population PD model established herein, the cumulative urine volume output following administration of the SC treatment, administered SC as 30 mg over the first hour and then as 12.5 mg per hour over the subsequent 4 hours (test treatment) was simulated. The observed vs simulated cumulative urine volume are shown in FIG. 3.

Population PD parameter values estimated from the established model fall into the 95%CI of the values estimated from the bootstrap replicates. Together with the diagnostic plots and CV values of the population PD parameter estimates, the final PD model is fully validated.

### Results

Results of the PK-PD modeling and simulation provide insight into understanding the relationship between the furosemide excretion rate into urine and the rate of urine volume output in patients with chronic heart failure. The IC₅₀ of 1,735,300 ng/hr suggests that following furosemide administration (e.g., oral, IV or SC), when the furosemide excretion rate in the urine reaches 1,735,300 ng/hr divided by the factor FER (the fraction of furosemide cleared/excreted from the renal pathway), 50% of the maximum rate of urine volume output will be reached.

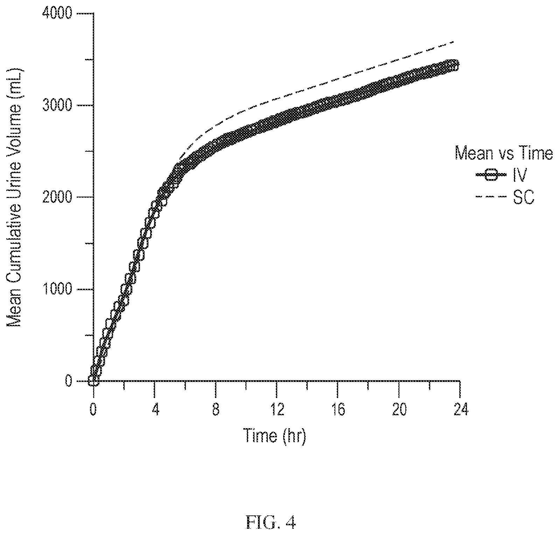

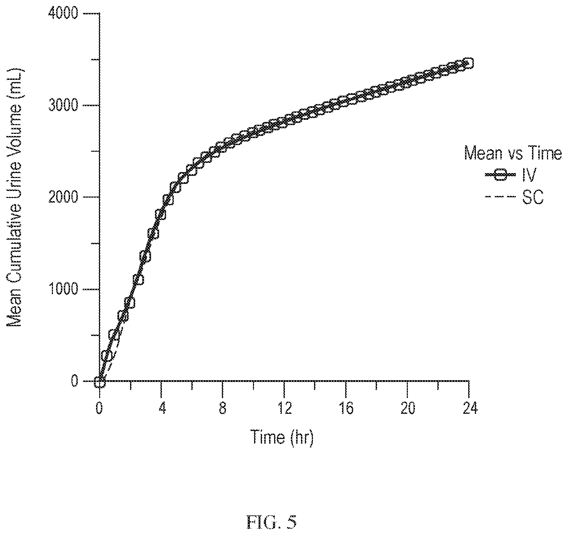

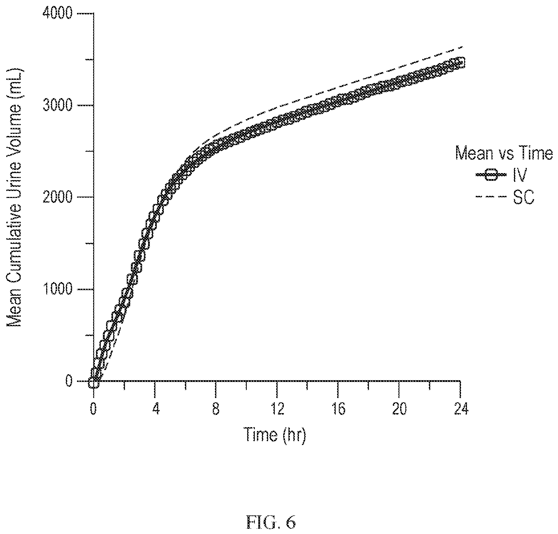

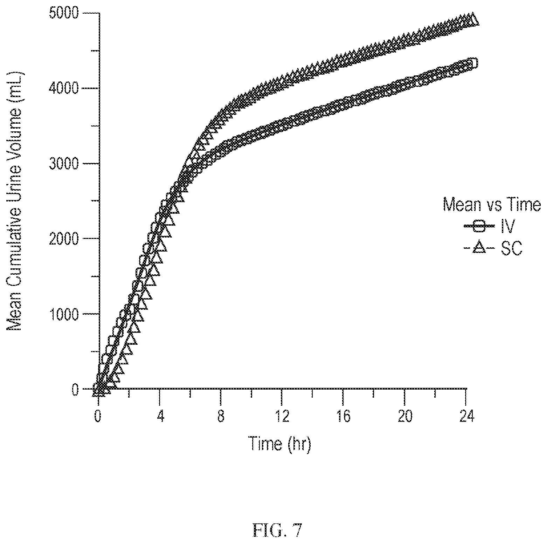

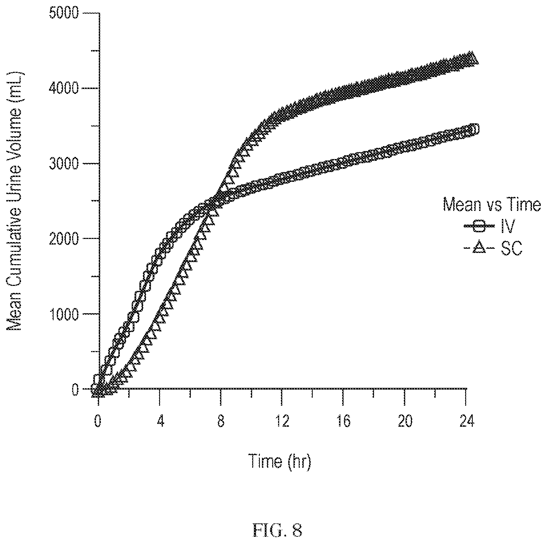

To simulate the cumulative urine volume output for 5 trials outlined in above, the model described herein was used (the exact value of FER is not needed).
- **Trial 1:** when 40 mg furosemide is subcutaneously administered at 2 timepoints, time 0 and 1.5 hr (over 30 minutes each time), the total urine volume output is similar to the IV treatment arm at 4 hours. By 6 hours to 24 hours post dose, the SC treatment ann produced ~250 mL urine volume more than the LD treatment arm. The results are depicted in FIG. 4.
- **Trial 2:** when 80 mg furosemide is subcutaneously administered over 1 hour, the total urine volume output is similar between the SC and IV treatment arms at all time points up to 24 hours. The results are depicted in FIG. 5.
- **Trial 3:** when 80 mg furosemide is subcutaneously administered over 2 hours, the total urine volume output from the SC treatment arm is similar to the IV treatment by 4 hours post dose and slightly greater than the IV treatment by 150 mL at 24 hours post dose. The results are depicted in FIG. 6.
- **Trial 4:** when 80 mg furosemide is subcutaneously administered over 4 hours, the total urine volume output from the SC treatment arm is similar to the IV treatment by 4 hours post dose. The SC treatment arm is estimated to produce 160 mL more than the urine volume output at 24 hours post dose compared to the IV treatment. The results are depicted in FIG. 7.
- **Trial 5:** when 80 mg furosemide is subcutaneously administered over 8 hours, the total urine volume output from the SC treatment is ~800 mL greater than the IV treatment by 4 hours post dose. However, the SC treatment ann is estimated to produce approximately 1000 mL more than the urine volume output at 24 hours post dose compared to the IV treatment. The results are depicted in FIG. 8.

Collectively, these trials demonstrated that when the same total dose of 80 mg was subcutaneously administered over 1, 24 and 8 hours in trials 1-5, by 8 hours post dose, the similar or slightly more total urine volume output is obtained compared to the IV treatment (40 mg administered over 2 minutes by IV bolus injection followed by a second dose of 40 mg over 2 minutes 120 minutes later). Further, as demonstrated in trial 5, when 80 mg furosemide is subcutaneously administered over 8 hours, the SC treatment arm is estimated to produce approximately 1000 mL (~30% increased) more than the urine volume output at 24 hours post dose compared to the IV treatment. This result is as expected as the furosemide excretion rate into urine remains above the IC₅₀ longer with increased infusion time used in trial 5 vs. trials 1-4 (8 hour versus 1, 2 and 4 hours, respectively).

### Example 3. Evaluation of the Impact of Duration of Subcutaneous Infusion of Furosemide Liquid Formulations (Furoscix^{®}) on Safety and Local Skin Tolerability

### Study Objective

Evaluate the safety and tolerability of shortened subcutaneous infusions of Furoscix (i.e., furosemide liquid formulation of Example 1).

### Methodology

An open label. non-randomized, single-center study.

### Number of Subjects

The number of subjects enrolled in the study was fifty.

### Criteria for Inclusion

To be enrolled in the study, subjects were required to meet the following inclusion criteria: an Institutional Review Board (IRB) approved informed consent was signed and dated prior to any study-related activities; male and female subjects 45 - 80 years of age; had the ability to understand the requirements of the study and willing to comply with all study procedures; and in the opinion of the Investigator, able to participate in the study.

### Criteria for Exclusion

A subject was not eligible to be enrolled in the study if any of the following criteria applied: receipt of loop diuretics (furosemide, bumetanide or torsemide) or thiazide diuretics (hydrochlorothiazide or metolazone) within 24 hours of study drug: receipt of potassium-sparing diuretics (spironolactone, amiloride) or oral calcium, potassium, or magnesium supplements (including multi-vitamins) within 24 hours of study drug; history of allergy to furosemide, sulfonamides, or other loop diuretics: any skin conditions currently requiring medical therapy; history of diabetes; presence or need for urinary catheterization, urinary tract abnormality or disorder interfering with urination; serum sodium < 130 mEq/L at screening; serum potassium ≤ 3.5 or ≥ 5.5 mEq/L at screening: serum magnesium < 1.5 or > 2.5 mEq/L at screening; serum chloride < 96 or > 106 mEq/L at screening; serum calcium < 8.5 or > 10.5 mg/dL at screening: estimated Glomerular Filtration Rate (GFR) < 30 mL/min/1.73 m2 by simplified Modification of Diet in Renal Disease (sMDRD) equation at screening: systolic BP (SBP) < 90 mmHg at screening or baseline: heart rate > 110 beats per minute (BPM) at screening or baseline; history of tinnitus or hearing impairment, including deafness: temperature > 38°C (oral or equivalent) at screening or baseline; blood alcohol concentration ≥ 2 mg/dL (0.02%) at screening or baseline: female subject who is pregnant or lactating; history of major abdominal surgery affecting the needle placement site: history of benign prostatic hyperplasia (BPH), prostatitis, or prostate cancer: urinary retention due to bladder emptying disorders and/or urethral narrowing; history of liver disease, cirrhosis, or ascites; any local abdominal skin condition on the day of treatment, i.e., sunburn, rash, eczema, etc.; allergies and/or sensitivities to adhesive bandages or medical tape: or any surgical or medical condition which in the opinion of the Investigator may interfere with participation in the study or which may affect the outcome of the study.

### Study Design

After signing informed consent, those subjects who met the inclusion/exclusion criteria including screening lab results were enrolled into the trial. Treatment began within 5 days of completion of the screening assessments. If the treatment visit did not occur within 5 days of the initial screening, subjects were rescreened, assigned a new subject number, and treated within 5 days of the rescreening.

There were 4 cohorts of subjects receiving the same dose of a subcutaneous (SC) infusion of Furoscix (furosemide injection 80 mg/10 mL) with varying infusion times. Furoscix was administered using the B. Braun Perfusor Space Infusion Pump via a B. Braun Omnifix 10 mL luer lock syringe connected to the Unomedical Neria^{™} infusion set (6 mm, 27 G, stainless steel needle for vertical insertion).

The initial cohort **(Cohort 1)** received a bi-phasic delivery over 5 hours, whereby 30 mg (3.75 mL) was infused over the first hour followed by 12.5 mg/hour (1.56 mL) over the subsequent 4 hours for the total dose of 80 mg (10mL) furosemide. Subsequent cohorts followed a stepwise decrease in infusion time, while maintaining the 80 mg/10 mL dose. Enrollment into subsequent cohorts began after completion of a safety assessment for each completed cohort by the Sponsor, Medical Monitor, and CRO. Cohorts 2a and 2b enrolled concurrently.

The infusion times for the subsequent cohorts were as follows:
**Cohort 2a:** continuous infusion of Furoscix 80 mg/10 mL over 2 hours
**Cohort 2b:** Furoscix 40 mg (5 mL) infused in the first 30 minutes: one-hour pause: remaining 40 mg (5 mL) infused over the last 30 minutes for a 2-hour total period.
**Cohort 3:** continuous infusion of Furoscix 80 mg/10 mL over 1 hour.
**Cohort 4:** continuous infusion of Furoscix 80 mg/10 mL over 30 minutes.

Subjects in each cohort were monitored for a minimum of 8 hours after the start of the infusion. Subjects were monitored for infusion site pain, obvious leakage, and adverse events. Injection site evaluation was performed, and blood pressure, heart rate, 12-lead ECG, and clinical labs were collected. Urine output and fluid intake were monitored for 8 hours after the start of the infusion.

A standardized meal with < 2 grams of sodium was provided. Water, juice, caffeine free beverages and low sodium snacks were permitted ad libitum. Caffeine intake was not permitted.

Subjects had a follow-up visit 24 - 48 hours after the start of the infusion to measure blood pressure and heart rate, conduct an inspection of the infusion site and assess all treatment-emergent AEs.

### Subject Disposition

Fifty (50) subjects were screened for the study. Forty-one (41) subjects were enrolled and included in the Intent to Treat (ITT) and Safety (SP) populations. Thirty-nine (39) subjects completed the study. Ten (10) subjects were excluded from the evaluable population (EP) due to leakage.

### Key Demographics

Key demographics of subjects in all populations (ITT, SP, EP) were similar across all cohorts. The average subject age per cohort ranged from 53.9 to 61.5 years. The average BMI per cohort ranged from 27.14 kg/m² to 29.10 kg/m². Gender distribution per cohort ranged from 25% to 62.5% male. Racial distribution per cohort ranged from 37.5% to 87.5% white: ethnicity ranged from 87.5% to 100% non-Hispanic.

### Duration of Treatment

Subjects received a Furoscix (80 mg/10 mL) infusion over 5 hours, 2 hours, 1 hour, or 30 minutes depending upon the assigned cohort. All subjects were monitored for 8 hours from the start of the infusion.

### Assessment of Endpoints

**Infusion Site Pain:** pain was assessed using an 11-point scale with assessments upon placement of the subcutaneous (SC) needle, upon activation of the SC infusion and periodically during the infusion, and post-infusion completion for 8 hours after the start of the infusion. Pain score was assessed on a scale from 0 to 10, where 0 is equivalent to no pain and 10 is equivalent to the worst possible pain.

Safety: the safety variables evaluated were incidence of treatment-emergent adverse events (TEAEs) and serious adverse events (SAEs) from the start of study drug administration through 24 to 48 hours after the start of the infusion.

Blood pressure, heart rate, 12-lead electrocardiogram (ECG) and clinical chemistry labs were collected during the study period.

**Exploratory Endpoint:** Total urine output was assessed for 8 hours after the start of the infusion. Subjects were instructed to void immediately prior to the start of the infusion, if possible, and this sample was discarded. Urine was collected from spontaneous voids, measured, recorded, and discarded for 8 hours after the start of the infusion.

### Statistical Methods

**Analysis Populations:** the following populations were used in the analysis:
Screened Population (ScP): All subjects who signed informed consent.
Intent to Treat Population (ITT): all subjects who signed informed consent and met eligibility criteria. This was equivalent to the enrolled population.
Safety Population (SP): all subjects who received study treatment. All safety and exploratory endpoints are summarized for the Safety Population.
Evaluable Population (EP): all subjects in the Safety Population who completed the infusion and experienced no evidence of obvious leakage. The exploratory endpoint for total urine output is summarized for the Evaluable Population.
   **Endpoints:** All endpoints were analyzed descriptively.
   Safety Analysis: Adverse Events were coded by Preferred Term (PT) and System Organ Class (SOC) using Medical Dictionary for Regulatory Activities (MedDRA) version 22.0. Listing of AEs was provided.

### Results

The majority of subjects experienced little or no pain during the procedure. The median pain score at needle placement was 0 (no pain) in all cohorts. During infusion, most subjects reported little or no pain. The post-infusion median pain score was 0. Pain scores were consistent across all cohorts (Table 1).

**Table 1. Infusion Site Pain at Needle Placement, During Infusion, and Post-Infusion**

| **Time Point** | **Pain Score** | **Cohort 1 N = 8** | **Cohort 2a N = 8** | **Cohort 2b N = 9** | **Cohort 3 N = 8** | **Cohort 4 N = 8** |
|---|---|---|---|---|---|---|
| Needle placement | | | | | | |
| | Median | 0.0 | 0.0 | 0.0 | 0.0 | 0.0 |
| | Q1, Q3 | 0.0, 0.0 | 0.0, 1.0 | 0.0, 0.0 | 0.0. 1.5 | 0.0, 1.5 |
| | Min, Max | 0,3 | 0,5 | 0,1 | 0,4 | 0,3 |

| During infusion^{a} | | | | | | |
|---|---|---|---|---|---|---|
| | Median | 0.0 | 0.0 | 0.0 | 0.0 | 0.5 |
| | Q1, Q3 | 0.0. 0.0 | 0.0, 0.0 | 0.0, 0.0 | 0.0, 2.5 | 0.0. 2.0 |
| | Min, Max | 0.2 | 0.6 | 0, 4 | 0,4 | 0,5 |

| Post-infusion^{a} | | | | | | |
|---|---|---|---|---|---|---|
| | Median | 0.0 | 0.0 | 0.0 | 0.0 | 0.0 |
| | Q1, Q3 | 0.0, 0.0 | 0.0, 0.0 | 0.0, 0.0 | 0.0, 0.0 | 0.0, 0.0 |
| | Min, Max | 0, 0 | 0, 0 | 0, 0 | 0, 0 | 0, 2 |

| | | | | | | |
|---|---|---|---|---|---|---|
| ^{a} Includes all pain scores reported during the time point. | | | | | | |

The total urine output over the 8-hour collection period was comparable across all cohorts (Tables 2 and 3).

**Table 2. Total Urine Output (Safety Population)**

| **Total Urine Output for 8 Hours (cc)** | **Cohort 1 N=8** | **Cohort 2a N = 8^{a}** | **Cohort 2b N = 9^{b}** | **Cohort 3 N = 8** | **Cohort 4 N=8** |
|---|---|---|---|---|---|
| Mean (SD) | 2623.8 (719.95) | 1990.0 (1113.75) | 2730.6 (1209.25) | 2868.8 (744.71) | 3141.3 (1204.21) |
| Median | 2280.0 | 2107.5 | 2800.0 | 3012.5 | 2990.0 |
| Q1, Q3 | 2115.0, 3255.0 | 1262.5, 2545.0 | 2275.0, 3400.2 | 2400.0. 3487.5 | 2362.5. 3722.5 |
| Min, Max | 1860, 3830 | 250.3840 | 50, 4430 | 1525, 3625 | 1600, 5380 |

| | | | | | |
|---|---|---|---|---|---|
| ^{a} Cohort 2a Subject 01-020 is discontinued due to AE. Total measured urine volume = 250 cc (1 collection). ^{b} Cohort 2b Subject 01-025 withdrew consent. Total measured urine volume = 50 cc (1 collection). | | | | | |

**Table 3. Total Urine Output (Evaluable Population^{a})**

| **Total Urine Output for 8 Hours (cc)** | **Cohort 1 N = 6** | **Cohort 2a N = 6^{b}** | **Cohort 2b N = 6** | **Cohort 3 N=5** | **Cohort 4 N = 6** |
|---|---|---|---|---|---|
| Mean (SD) | 2858.3 (678.30) | 2315.8 (997.21) | 2945.8 (808.59) | 3070.0 (530.74) | 3571.7 (1056.37) |
| Median | 2762.5 | 2332.5 | 2762.5 | 3350.0 | 3122.5 |
| Q1, Q3 | 2260.0, 3285.0 | 2065.0, 2575.0 | 2275.0, 3195.0 | 2675.0, 3375.0 | 2880.0, 4300.0 |
| Min, Max | 2250, 3830 | 750, 3840 | 2250, 4430 | 2350. 3600 | 2625. 5380 |

| | | | | | |
|---|---|---|---|---|---|
| ^{a} The Evaluable Population includes all subjects in the Safety Population who completed the infusion and experienced no evidence of obvious leakage. ^{b} Cohort 2a Subject 01-021 missed urine collection (voided in toilet). Total measured urine volume = 750 cc. | | | | | |

**Safety Results:** There were forty (40) adverse events experienced by twenty-four (24) subjects. All events were assessed to be mild or moderate, and non-serious. There were no overt safety issues observed, and no reported deaths.

No apparent changes were observed in serum chemistry laboratory values, vital signs, or ECG parameters in any of the cohorts.

### Conclusions

The majority of subjects experienced little or no pain during and after the subcutaneous infusion of Furoscix. Pain scores were consistent across all cohorts.

No oven safety issues were observed in any of the cohorts.

The total urine output over the 8-hour collection period was comparable across all cohorts.

## Claims

1. A liquid pharmaceutical formulation comprising 80 mg furosemide in a total volume of 10 mL for use in treating a condition selected from the group consisting of congestion due to fluid overload, edema, and hypertension, in a human in need thereof, comprising administering subcutaneously to the human a total volume of 10 mL of the liquid pharmaceutical formulation over a period of 0.5 hours ±10% to 2 hours ±10%, wherein during administration of the liquid pharmaceutical formulation and/or nearly immediately after administration of the liquid pharmaceutical formulation is complete, the administration results in a pain score of 0, wherein the pain score is measured using an 11-point numeric pain assessment scale.

2. The liquid pharmaceutical formulation for use according to claim 1, wherein about 2 hours after administration is initiated, a total urine output of about 450 mL to about 950 mL results.

3. The liquid pharmaceutical formulation for use according to claim 1 or 2, wherein about 6 hours after administration is initiated, a total urine output of about 2100 mL to about 2600 mL results.

4. The liquid pharmaceutical formulation for use according to any one of claims 1-3, wherein about 24 hours after administration is initiated, a total urine output of about 3200 mL to about 4200 mL results.

5. The liquid pharmaceutical formulation for use according to any one of claims 1-4, wherein about 2 hours after administration is initiated, a total urine sodium excretion of about 40 mmol to about 110 mmol results.

6. The liquid pharmaceutical formulation for use according to any one of claims 1-5, wherein about 6 hours after administration is initiated, a total urine sodium excretion of about 220 mmol to about 300 mmol results.

7. The liquid pharmaceutical formulation for use according to any one of claims 1-6, wherein about 24 hours after administration is initiated, a total urine sodium excretion of **about 350 mmol to about 480 mmol results.**

8. The liquid pharmaceutical formulation for use according to any one of claims 1-7, wherein administering comprises a continuous delivery profile over a period of about two hours.

9. The liquid pharmaceutical formulation for use according to any one of claims 1-7, wherein administering comprises a bi-phasic delivery profile over a period of about two hours.

10. The liquid pharmaceutical formulation for use according to any one of claims 1-7, wherein administering comprises a continuous delivery profile over a period of about 1.5 hours.

11. The liquid pharmaceutical formulation for use according to any one of claims 1-7, wherein administering comprises a continuous delivery profile over a period of about one hour.

12. The liquid pharmaceutical formulation for use according to any one of claims 1-7, wherein administering comprises a continuous delivery profile over a period of about 0.5 hours.

13. The liquid pharmaceutical formulation for use according to any one of claims 1-12, wherein the liquid pharmaceutical formulation is administered from a prefilled polymeric cartridge, optionally wherein the prefilled polymeric cartridge is associated with an on-body wearable delivery device.

14. The liquid pharmaceutical formulation for use according to claim 13, wherein the polymeric prefilled cartridge is a single-use cartridge.

15. The liquid pharmaceutical formulation for use according to any one of claims 1-14, wherein the edema is associated with congestive heart failure, cirrhosis of the liver or renal disease, optionally wherein the renal disease is nephrotic syndrome.

## Patentansprüche

1. Flüssige pharmazeutische Formulierung, umfassend 80 mg Furosemid in einem Gesamtvolumen von 10 ml zur Verwendung bei der Behandlung eines Zustands, ausgewählt aus der Gruppe, bestehend aus Stauung aufgrund von Fluidüberlastung, Ödemen und Bluthochdruck, bei einem Menschen, der dies benötigt, umfassend die subkutane Verabreichung eines Gesamtvolumens von 10 ml der flüssigen pharmazeutischen Formulierung an den Menschen über einen Zeitraum von 0-5 Stunden ±10 % bis 2 Stunden ±10 %, wobei während der Verabreichung der flüssigen pharmazeutischen Formulierung und/oder nahezu unmittelbar nach Abschluss der Verabreichung der flüssigen pharmazeutischen Formulierung die Verabreichung zu einem Schmerzwert von 0 führt, wobei der Schmerzwert unter Verwendung einer numerischen 11-Punkte-Schmerzbewertungsskala gemessen wird.

2. Flüssige pharmazeutische Formulierung zur Verwendung nach Anspruch 1, wobei etwa 2 Stunden nach Beginn der Verabreichung eine Gesamturinausscheidung von etwa 450 ml bis etwa 950 ml resultiert.

3. Flüssige pharmazeutische Formulierung zur Verwendung nach Anspruch 1 oder 2, wobei etwa 6 Stunden nach Beginn der Verabreichung eine Gesamturinausscheidung von etwa 2100 ml bis etwa 2600 ml resultiert.

4. Flüssige pharmazeutische Formulierung zur Verwendung nach einem der Ansprüche 1 bis 3, wobei etwa 24 Stunden nach Beginn der Verabreichung eine Gesamturinausscheidung von etwa 3200 ml bis etwa 4200 ml resultiert.

5. Flüssige pharmazeutische Formulierung zur Verwendung nach einem der Ansprüche 1 bis 4, wobei etwa 2 Stunden nach Beginn der Verabreichung eine Gesamturinnatriumausscheidung von etwa 40 mmol bis etwa 110 mmol resultiert.

6. Flüssige pharmazeutische Formulierung zur Verwendung nach einem der Ansprüche 1 bis 5, wobei etwa 6 Stunden nach Beginn der Verabreichung eine Gesamturinausscheidung im Urin von etwa 220 mmol bis etwa 300 mmol resultiert.

7. Flüssige pharmazeutische Formulierung zur Verwendung nach einem der Ansprüche 1 bis 6, wobei etwa 24 Stunden nach Beginn der Verabreichung eine Gesamturinnatriumausscheidung von etwa 350 mmol bis etwa 480 mmol resultiert.

8. Flüssige pharmazeutische Formulierung zur Verwendung nach einem der Ansprüche 1 bis 7, wobei die Verabreichung ein kontinuierliches Abgabeprofil über einen Zeitraum von etwa zwei Stunden umfasst.

9. Flüssige pharmazeutische Formulierung zur Verwendung nach einem der Ansprüche 1 bis 7, wobei die Verabreichung ein zweiphasiges Abgabeprofil über einen Zeitraum von etwa zwei Stunden umfasst.

10. Flüssige pharmazeutische Formulierung zur Verwendung nach einem der Ansprüche 1 bis 7, wobei die Verabreichung ein kontinuierliches Abgabeprofil über einen Zeitraum von etwa 1,5 Stunden umfasst.

11. Flüssige pharmazeutische Formulierung zur Verwendung nach einem der Ansprüche 1 bis 7, wobei die Verabreichung ein kontinuierliches Abgabeprofil über einen Zeitraum von etwa einer Stunde umfasst.

12. Flüssige pharmazeutische Formulierung zur Verwendung nach einem der Ansprüche 1 bis 7, wobei die Verabreichung ein kontinuierliches Abgabeprofil über einen Zeitraum von etwa 0,5 Stunden umfasst.

13. Flüssige pharmazeutische Formulierung zur Verwendung nach einem der Ansprüche 1 bis 12, wobei die flüssige pharmazeutische Formulierung aus einer vorgefüllten polymeren Patrone verabreicht wird, wobei die vorgefüllte polymere Patrone optional mit einer am Körper tragbaren Abgabevorrichtung verbunden ist.

14. Flüssige pharmazeutische Formulierung zur Verwendung nach Anspruch 13, wobei die polymere vorgefüllte Patrone eine Einwegpatrone ist.

15. Flüssige pharmazeutische Formulierung zur Verwendung nach einem der Ansprüche 1 bis 14, wobei das Ödem mit kongestiver Herzinsuffizienz, Leberzirrhose oder einer Nierenerkrankung zusammenhängt, wobei die Nierenerkrankung optional das nephrotische Syndrom ist.

## Revendications

1. Formulation pharmaceutique liquide comprenant 80 mg de furosémide dans un volume total de 10 ml destinée à être utilisée dans le traitement d'une affection sélectionnée dans le groupe constitué de la congestion due à une surcharge liquidienne, de l'œdème, et de l'hypertension, chez un être humain en ayant besoin, comprenant l'administration sous-cutanée à l'être humain d'un volume total de 10 ml de la formulation pharmaceutique liquide sur une période de 0,5 heure ± 10 % à 2 heures ± 10 %, dans laquelle, pendant l'administration de la formulation pharmaceutique liquide et/ou presque immédiatement après la fin de l'administration de la formulation pharmaceutique liquide, l'administration entraîne un score de douleur de 0, dans laquelle le score de douleur est mesuré à l'aide d'une échelle numérique d'évaluation de la douleur à 11 points.

2. Formulation pharmaceutique liquide destinée à être utilisée selon la revendication 1, dans laquelle environ 2 heures après le début de l'administration, un débit urinaire total d'environ 450 ml à environ 950 ml est obtenu.

3. Formulation pharmaceutique liquide destinée à être utilisée selon la revendication 1 ou 2, dans laquelle environ 6 heures après le début de l'administration, un débit urinaire total d'environ 2100 ml à environ 2600 ml est obtenu.

4. Formulation pharmaceutique liquide destinée à être utilisée selon l'une quelconque des revendications 1 à 3, dans laquelle environ 24 heures après le début de l'administration, un débit urinaire total d'environ 3200 ml à environ 4200 ml est obtenu.

5. Formulation pharmaceutique liquide destinée à être utilisée selon l'une quelconque des revendications 1 à 4, dans laquelle environ 2 heures après le début de l'administration, une excrétion urinaire totale de sodium d'environ 40 mmol à environ 110 mmol est obtenue.

6. Formulation pharmaceutique liquide destinée à être utilisée selon l'une quelconque des revendications 1 à 5, dans laquelle environ 6 heures après le début de l'administration, une excrétion urinaire totale de sodium d'environ 220 mmol à environ 300 mmol est obtenue.

7. Formulation pharmaceutique liquide destinée à être utilisée selon l'une quelconque des revendications 1 à 6, dans laquelle environ 24 heures après le début de l'administration, une excrétion urinaire totale de sodium d'environ 350 mmol à environ 480 mmol est obtenue.

8. Formulation pharmaceutique liquide destinée à être utilisée selon l'une quelconque des revendications 1 à 7, dans laquelle l'administration comprend un profil d'administration continue sur une période d'environ deux heures.

9. Formulation pharmaceutique liquide destinée à être utilisée selon l'une quelconque des revendications 1 à 7, dans laquelle l'administration comprend un profil d'administration biphasique sur une période d'environ deux heures.

10. Formulation pharmaceutique liquide destinée à être utilisée selon l'une quelconque des revendications 1 à 7, dans laquelle l'administration comprend un profil d'administration continue sur une période d'environ 1,5 heure.

11. Formulation pharmaceutique liquide destinée à être utilisée selon l'une quelconque des revendications 1 à 7, dans laquelle l'administration comprend un profil d'administration continue sur une période d'environ une heure.

12. Formulation pharmaceutique liquide destinée à être utilisée selon l'une quelconque des revendications 1 à 7, dans laquelle l'administration comprend un profil d'administration continue sur une période d'environ 0,5 heure.

13. Formulation pharmaceutique liquide destinée à être utilisée selon l'une quelconque des revendications 1 à 12, dans laquelle la formulation pharmaceutique liquide est administrée à partir d'une cartouche polymère préremplie, éventuellement dans laquelle la cartouche polymère préremplie est associée à un dispositif d'administration portable sur le corps.

14. Formulation pharmaceutique liquide destinée à être utilisée selon la revendication 13, dans laquelle la cartouche préremplie polymère est une cartouche à usage unique.

15. Formulation pharmaceutique liquide destinée à être utilisée selon l'une quelconque des revendications 1 à 14, dans laquelle l'œdème est associé à une insuffisance cardiaque congestive, une cirrhose du foie ou une maladie rénale, éventuellement dans laquelle la maladie rénale est un syndrome néphrotique.
